# EUROPEAN PATENT SPECIFICATION

(11) **EP 2 183 357 B1**
(45) Date of publication and mention of the grant of the patent: **13.04.2016**
(21) Application number: 08795050.7
(22) Date of filing: 06.08.2008
(51) Int. Cl.: C12N 5/00

(54) **TISSUE ORGANIZING STRUCTURE AND THERAPEUTIC METHODS**
GEWEBEORGANISATIONSSTRUKTUR UND THERAPEUTISCHE VERFAHREN
STRUCTURE D'ORGANISATION TISSULAIRE ET PROCÉDÉS THÉRAPEUTIQUES

(30) Priority: 06.08.2007 US 963606 P; 10.12.2007 US 5930
(43) Date of publication of application: 12.05.2010
(73) Proprietor: Southern Connecticut State University, New Haven, CT 06515 (US)
(72) Inventor: CRAWFORD, Sarah, C., Milford, CT 06460 (US); POTTER, Herbert, G., Hamden, CT 06518 (US); HERBERT, Tasino, D., Hamden, CT 06514 (VG)
(74) Representative: Glawe, Delfs, Moll
(86) International application number: PCT/US2008/009419
(87) International publication number: WO 2009/020604

(56) References cited:
- WO-A2-2005/121369
- US-A1- 2004 028 692
- US-A1- 2005 138 692
- CRAWFORD SARAH C ET AL: "Novel extracts from diverse primitive plant species block solid tumor formation and induce tumor cell death: role of focal adhesion kinase (FAK) and actin cytoskeleton" PROCEEDINGS OF THE ANNUAL MEETING OF THE AMERICAN ASSOCIATION FOR CANCER RESEARCH, AMERICAN ASSOCIATION FOR CANCER RESEARCH, US, vol. 48, 1 April 2007 (2007-04-01), page 1309, XP001538898 ISSN: 0197-016X
- BEAUDOIN A R ET AL: "Shedding of vesicular material from the cell surface of eukaryotic cells: different cellular phenomena" BIOCHIMICA ET BIOPHYSICA ACTA. MR. REVIEWS ON BIOMEMBRANES, ELSEVIER, AMSTERDAM, NL LNKD- DOI:10.1016/0304-4157(91)90014-N, vol. 1071, no. 3, 13 November 1991 (1991-11-13), pages 203-219, XP023580336 ISSN: 0304-4157 [retrieved on 1991-11-13]
- MONIKA BAJ-KRZYWORZEKA ET AL: "Tumour-derived microvesicles carry several surface determinants and mRNA of tumour cells and transfer some of these determinants to monocytes" CANCER IMMUNOLOGY, IMMUNOTHERAPY, SPRINGER, BERLIN, DE LNKD- DOI:10.1007/S00262-005-0075-9, vol. 55, no. 7, 1 July 2006 (2006-07-01), pages 808-818, XP019333260 ISSN: 1432-0851
- VALADI H ET AL: "Exosome-mediated transfer of mRNAs and microRNAs is a novel mechanism of genetic exchange between cells" NATURE CELL BIOLOGY, NATURE PUBLISHING GROUP, GB LNKD- DOI:10.1038/NCB1596, vol. 9, no. 6, 6 June 2007 (2007-06-06), pages 654-659, XP003016816 ISSN: 1465-7392
- JANOWSKA-WIECZOREK ANNA ET AL: "Microvesicles derived from activated platelets induce metastasis and angiogenesis in lung cancer" INTERNATIONAL JOURNAL OF CANCER, vol. 113, no. 5, 20 February 2005 (2005-02-20), pages 752-760, XP002592724 ISSN: 0020-7136
- WYSOCZYNSKI MARCIN ET AL: "Lung cancer secreted microvesicles: Underappreciated modulators of microenvironment in expanding tumors" INTERNATIONAL JOURNAL OF CANCER, vol. 125, no. 7, October 2009 (2009-10), pages 1595-1603, XP002592725 ISSN: 0020-7136
- CLARK ET AL.: 'Annexins of Plant Cells.' PLANT PHYSIOL. vol. 109, 01 December 1995, pages 1133 - 1139, XP002458002
- FROISSARD ET AL.: 'Novel Secretory Vesicle Proteins Essential for Membrane Fusion Display Extracellular-Matrix Domains.' TRAFFIC vol. 5, 01 May 2004, pages 493 - 502, XP008130212
- MILLIMAGGI ET AL.: 'Tumor Vesicle Associated CD147. Modulates the Angiogenic Capability of Endothelial Cells.' NEOPLASIA vol. 9, no. 4, 01 April 2007, pages 349 - 357, XP008130129
- HUGEL ET AL.: 'Membrane Microparticles: Two Sides of the Coin.' PHYSIOLOGY vol. 20, 01 February 2005, pages 22 - 27, XP002573862

## Description

### CROSS-REFERENCE TO RELATED APPLICATIONS

This application claims priority from U.S. Provisional Patent Application No. 60/963,606 filed August 6,. 2007, and U.S. Provisional Patent Application No. 61/005,930 filed December 10, 2007.

### BACKGROUND

Despite many advances and current progress in the early detection of cancer, many malignant tumors are only first diagnosed at late stages of disease, long after the early events responsible for tumor development have given way to advanced, and often, widespread metastatic disease. This unfortunate circumstance both complicates cancer therapeutics and obfuscates a major objective of cancer biology: to explore the fundamental early stage events responsible for malignant tumor development and spread in the body. Research studies designed to recapitulate early stages in the natural history of solid tumor formation and development might be expected to afford a unique opportunity to assess fundamental parameters that underscore the abnormal growth behavior of genetically transformed cells and also to provide key insights into fundamental, but as yet, poorly understood oncogenic behaviors, including mechanisms of solid tumor formation, invasiveness and spread that distinguish cancer cells from their normal counterparts.

Identification of a novel cell-derived structure, hereinafter referred to as "tissue organizing structure", or "TOS", and methods for using TOS are described herein. TOS possesses unique functional capabilities whose clinical applications include, but are not limited to, tissue generation from individual cellular components, tissue morphogenesis, gene transfer therapeutics, and use as a novel target or therapeutic agent for cancer prevention and therapeutics.

The existence of these novel cellular components were previously unidentified in multi-cellular systems. These unusual structures were discovered during approximately thirty hours of live cell microscopy studies of tumor cells cultured under conditions designed to promote the formation of multi-cellular microscopic tumors. TOS play a direct role in orchestrating the process of solid tumor development in tumor cell lines from human malignancies of the colon, breast, lung and brain, which comprise some of the most common human cancers. The migrations and extensions of tumor masses resulting from TOS interactions suggest that they play a critical role in processes associated with tumor formation, with tumor spread and metastasis, and with fundamental components of cancer growth.

In addition, the first documentation is presented herein of properties associated with TOS comprising tissue organization, genetic recombination, cell transformation, phenotypic reversion and solid tumor behavior and structural organization that define the utility of TOS as bio-medically useful agents. The TOS have now been identified, their cellular origins and properties documented, and several clinical applications assessed which have not been done in any prior study. Additional biological parameters involve tissue morphogenetic and therapeutic properties associated with TOS activity enabling phenotypic cell transformation and the phenotypic reversion and induction of cell death in human cancers. Additional biological parameters describe TOS in primitive plant species that possess anti-cancer activity.

### Description of Related Art

Over the past few years, increasing attention has been given to the heterogeneous components of the microenvironment in which cancers develop. Research studies have identified different classes of tumor cells including stem cell components that may serve as the drivers of malignant growth (see, for example, Wicha et al. Cancer Res. 66(4): 1883-90 (2006); Ferretti et al. Hum Reprod Update. 13(2): 121-41 (2007)). In addition, the tissue stroma and its effects on tumorgenesis have been an important object of study (see, for example, Tang et al. FASEB J.21(14): 3777-85 (2007)). The role of abnormal vascularization associated with tumor-initiated angiogenesis has been shown to be instrumental in tumor spread and disease progression (see, for example, Timmins et al. Angiogenesis 7(2):97-103 (2004)). This complexity of the tumor microenvironment further complicates attempts to assess fundamental processes driving the process of solid tumor development and spread.

Historically, the tissue culture system has played a critical role in the study of cancer, in exploring cancer cell genetics, the transformed phenotype, chemical and viral carcinogenic mechanisms and preclinical therapeutic assessments. The studies conducted herein to explore the early stages of solid tumor development have utilized the tissue culture system to replicate various aspects of tumor cell behaviors that may be applicable to the natural setting in which tumors develop. Previous studies have documented that tumor cells derived from many diverse types of human malignancies can be induced to form solid tumor masses when deprived of a suitable substrate for attachment, thereby preventing monolayer culture growth in vitro. (see, for example, Sutherland, Science. 240: 177-184 (1988); Kunz-Schughart et al. Inf. J .Exp. Pathol. 79: 1-23 (1998); Santini and Rainaldi, Pathobiology. 67(3): 148-57 (1999); and Santini et al. Crit Rev Oncol Hematol. 36(2-3):75-87 (2000)). Under these conditions, individual tumor cells spontaneously aggregate to form solid tumor masses that have been termed "multi-cellular tumor spheroids" (MTS). More recently, in vitro studies of MTS have been expanded to include additional components of the tumor microenvironment, including endothelial, immunological and connective tissue components in an attempt to define the relative contributions of each of these micro-environmental factors to solid. tumor behavior. (see, for example, Drasdo and H6hme, Phys Biol. 2(3): 133-47 (2005) and Delsanto et al., Phys Rev Lett. 94(14): 148105 (2005)). Extensive studies of the in vitro solid tumor model suggest that it bears some similarity to microscopic solid tumor lesions in vivo and that cultured MTS respond to cancer therapeutics more similarly to solid tumors in vivo than do two-dimensional monolayer cultures of tumor cells. (see, for example, Song et al., In Vitro Cell Dev. Biol Anim. 40 (8,9):262-267 (2004)). More recently, tumor spheroids have been used to model chemotherapy responses to positron emission tomography (PET) imaging studies (see, for example, Monazzam A, et al. Application of the multi-cellular tumor spheroid model to screen PET tracers for analysis of early response of chemotherapy in breast cancer. Breast Cancer Research (9:45)(2007)).

There are many important theoretical models that have been proposed to explain the biological behavior of tumor cells relevant to the processes driving solid tumor formation, increases in mass, and invasive and expansive properties that may contribute to the metastatic phenotype. Many of these models are based on studies of solid tumor multi-cellular spheroids (MTS) in vitro. An early model proposed by Landry et al., used mathematical expressions to correlate tumor growth rate with specific measurable growth parameters, and predicted a linear expansion of spheroid diameter with time. (see, for example, Landry et al., Cell Tissue Kinet. 15 (6):585-94 (1982)).

A more recent model designed to explain solid tumor growth behavior involved the analysis of nutrient and biomechanical forces to assess the spatio-temporal growth dynamics of solid tumor cultures. (see, for example, Tindall and Please, Bull Math Biol 69(4): 1147-1165 (2007)). These computer-aided modeling studies suggested that a bio-mechanically mediated form of growth inhibition may be responsible for transitions from exponential to sub-exponential growth rates in large size tumor spheroids. Another recently proposed mathematical model was based on energy metabolism and reaction/diffusion equations to predict growth rate patterns and regions of quiescence within solid tumors that might have predictive value in establishing critical parameters of tumor survival. (see, for example, Venkatasubramanian et al., J Theor Biol. 242(2):440-53 (2006)). Additional studies of tumor cell growth dynamics associated with spheroid formation of a glioblastoma multiforme cell line based on physical measurements of spatio-temporal cell distributions, immunohistochemistry, and flow cytometry indicated that simple exponential growth kinetics could not account for the growth behavior characteristic of tumor spheroid formation, (see, for example, Stein et al., Biophys J. 92(1):356-65 (2007)).

The growth pattern of microscopic glioblastoma multiforme was studied in vitro and observed that invasive tumor cells organize in branches. A model was proposed suggesting that homotypic attractions defined as the tendency of cells to travel previous paths coupled with heterotypic chemotaxis as the basis for the formation of these invasive zones (see, for example, Sander and Deisboeck, Phys Rev E Stat Nonlin Soft Matter Phys. 66 (5 Pt 1):051901 (Nov., 2002) and Khain and Sander, Phys Rev Lett 96(18): 188103 (2006)). In addition, it has been proposed that volumetric growth dynamics in solid tumors may depend on surface extension parameters based on the "'universal scaling law" (see, for example, Doisboeck et al., Med Hypotheses. 67(6); 1338-41 (2006)). They propose that cancer invasion should occur via a few branches of mobile cells and that this surface expansion may permit the diffusion of nutrients to permit tumor establishment in the absence of neoangiogenesis. Scaling techniques utilized to assess the fractal nature of developing tumors from cell lines in vitro and also in vivo have shown that both groups of tumors display similar growth dynamics which correspond to molecular beam epitaxy (MBE) universality class. (see, for example, Bru et al., Biophys J. 85(5):2948-61 (2003)). MBE dynamics are characterized by a linear growth rate, limitation of cell proliferation to the edge or border of the tumor and the surface diffusion of cells at the growing edge, in place of exponential growth behavior. The authors proposed that a significant determinant of cell movements or diffusion at the tumor border involves the intrinsic requirement of the tumor for space in competition with normal host tissue, perhaps more important than nutrient or other factors in driving this expansion. Their experimental data suggest that cell surface diffusion represents a critical determinant governing the pattern of tumor growth. These arguments provide conceptual support for TOS being the intrinsic cellular source of tumor expansion and invasiveness that permits the development of this system consistent with this theoretical framework.

Recent studies of solid tumor micro-environmental associations in breast cancers and other solid tumors have led to the proposed concept of an epithelial-mesenchymal transition (EMT) as the basis for many of the morphological cell structure changes associated with oncogenesis of epithelial cells to abnormal cells of fibroblastic morphology. (see, for example, Kokkinos et al., Cells Tissues Organs. 185 (1-3): 91-203 (2007)). Moreover, genetic analyses of normal stromal tissue in contact with malignant colon cells suggest that EMS may also affect the tumor microenvironment and promote disease spread as the result of genetic induced in-tumor associated stroma. (see, for example, Sheehan et al., Oncogene. 27(3):323-31 (Jan. 10, 2008)).

Our findings suggest a mechanism by which this proposed "crosstalk" might occur, in that TOS fusions with normal stromal cells surrounding a developing tumor could induce genetic changes consistent with these observations. Research data supportive of this model conducted in this laboratory comprises: (1) phenotypic transformation of normal cultured lung cells (cell line WI38) to a highly aggressive malignant cell type by cell-free transfer of TOS isolated from a malignant lung tumor cell line (cell line H1299) (2) phenotypic reversion and cell death induction of malignant lung tumor cell line (cell line H1299) by normal lung fibroblasts (cell line WI38); and (3) morphological evidence of EMT-like tissue transformations induced by TOS from breast tissue incubated with glial brain tissue.

Additional theories have suggested that cell fusions between genetically transformed "pre-malignant" cells and tissue stem cells may mediate horizontal gene transfers responsible for early stage oncogenesis (see, for example, Tysnes and Bjerkvig, Biochem.Biophys.Acta. 1775(2): 283-97 (June, 2007)). However, the observations herein provide direct evidence that cell fusions and horizontal genetic recombinations occur frequently in early stages of solid tumor formation in vitro and that TOS cell components are the structural vehicle responsible for these events.

Other journal publications include a recently published study by Nedawi et al (2008) that discusses the role of secreted membrane vesicles called exosomes derived from mast cells in inducing plasminogen activator inhibitor type I activity in endothelial cells. (see, for example, "Intercellular transfer of the oncogenic receptor EGFRvIII by microvescicles derived from tumor cells", Al-Nedawi et al, NCB 10(5) (May 2008)). The findings in the Medawi study differ substantively from the TOS discovery in that the researchers conducted the study using conventional exosomes, performed the study as an in vitro experiment with speculative biological significance in an intact cellular system, and postulated that the resultant phenotypic changes were based on passive protein transfer rather than resulting from a stable morphogenetic transformation, an activity that is documented in TOS particles. An earlier study by Bergsmedh et al (2001) documented the horizontal transfer of oncogenes by apoptotic bodies. (see for example, "Horizontal Transfer of Oncogenes by Uptake of Apoptotic bodies" Bergdmedh et al., PNAS 98(11) (May 2001)). This study also differed substantively from the TOS finding in that apoptotic bodies obtained from dead cells were used as a source of gene transfer. In addition, genetically altered cell lines were used as a source of transfected genes for horizontal transfer. Most importantly, the gene transfer and associated phenotypic effects were unstable, inducing a transient phenotypic alteration that resulted in loss of the transferred genes within four weeks in culture. In contrast, the TOS activities are associated with a stable morphogenetic transformation resulting in the production of stable transformed cell lines that have been maintained for many months in culture. Yet another study, del Conde et al Blood 106 (March, 2005) documented the capacity of monocyte/macrophage derived microvesicles containing tissue factors to fuse with activated platelets to initiate platelet coagulation. The demonstrated biomedical activities of TOS illustrate the clinical potential of, and need for, TOS as a diagnostic and treatment agent in human health and disease.

The related art does not address the identification of TOS nor its biomedical significance as identified herein. The identification of TOS and the methods of utilization are a significant departure from the prior art in that novel processes have been identified by which cancer cells organize in culture to form three-dimensional solid tumors that involves cell-derived membrane-bound structures (TOS) that orchestrate the process of solid tumor formation and expansion.

An embodiment of the process for TOS mediated solid tumor development comprises a functional resemblance to other biological systems, which thus has relevance to cancer biology, including the blastocyst stage of embryogenesis which appears to resemble the fission-like process associated with TOS formation; schizogamy, which is a rapid cell division of the malarial parasite in the liver, and resembles the fission-like reproduction of TOS; and simple genetic exchange mechanisms characteristic of unicellular organisms. These comparisons may be significant since oncogenesis may recapitulate in early stage embryogenic stem cell developmental processes, and may also occur in a parasitic mode reminiscent of primitive eukaryotic parasitic systems.

### SUMMARY

A unique cellular component with unprecedented and previously undescribed properties with direct clinical and biomedical therapeutic applications is described herein. No previous study has identified or characterized the activities or biomedical uses of the unique cellular component designated herein as "tissue organizing structure" or "TOS". Nor has any previous study documented the specific activities of the TOS applicable to the prevention and treatment of diseases, including cancer, as well as tissue engineering and genetic engineering for the treatment or prevention of diseases. US-A-2004/028692 discloses a process for sensitizing antigen-presenting cells, in particular dendritic cells, means for the implementation of the process and membrane vesicles, i.e. texosomes and dexosomes, having an immunogenic potency. In contrast to said prior art, the compositions of the present invention as defined in the claims comprise novel tissue organizing structure (TOS), wherein said TOS is a eukaryotic cell-derived, cell-free membrane-delimited vesicle, has a diameter about 2 microns and an interior comprising DNA and is capable of inducing stable morphogenetic transformation of recipient cells, which is not known or anticipated from said prior art. The invention is set out in the appended claims. The embodiments of the description which do not fall within the scope of said claims are provided for illustrative purposes only and do not form part of the present invention.

### Identification and Characterization of TOS

The spatio-dynamic data that were obtained from microscopic observations of live tumor imaging and staining for structural assessment indicated that one embodiment of TOS formation is as a "bud" from the surface membrane of tumor cells. Individual TOS budding is generally induced by cell detachment from substrate or from other cell contacts, indicating that an important stimulus for TOS production is the abrogation of cell-to-substrate or cell-to-cell contacts and associated signaling pathways. Thus TOS appear to be membrane-delimited vesicles that frequently contain genetic material based on their staining properties, although empty vesicles that do not contain genetic material have also been observed.

A plurality of TOS are produced by a novel autonomously driven reproduction mechanism occurring via rapid cell fission. TOS-cell interactions were observed to induce the formation of large numbers of TOS vesicles within the targeted cell, which was subsequently associated with cell lysis and release of the TOS vesicles. This TOS-induced production of TOS occurs via a fission-like process in which a cell is induced by TOS-mediated activity to undergo a rapid morphogenesis to what is observed to be a plurality of TOS within the cell, or "bag of TOS", that are subsequently released by the rupture or lysis of the cell membrane.

One or more methods of extracting TOS from cells and enabling isolation and purification of TOS as a cell-free extract are provided herein. A method utilizing cells of human origin comprises generally culturing cells to stimulate TOS production, treating the cells with trypsin to accumulate the TOS, the media and cells are then removed from the culture dish to isolate the TOS, the TOS are scraped off the bottom off the dish and re-suspended in culture medium for removal in pure form in the absence of cellular material. A method utilizing plant cells comprises generally preparing plant material (leaves and stems) and then culturing as above to isolate and purify TOS.

One or more methods of identification of TOS are provided herein generally comprising culturing cancer cell lines, utilizing videography and/or photomicroscopy to record tumor chronology and development, and identification of TOS by direct observation and/or selective staining techniques.

One or more methods of detection of TOS in a biological sample are provided herein. TOS may be routinely identified in cells in culture following trypsinization and replating. This method of cell culture manipulation for routine passage and maintenance induces the spontaneous budding of TOS vesicles from the surface of the cultured cell. Confluent substrate attached cells are not generally associated with TOS formation or release from cultured cells.

The mechanism of action of these unique TOS cellular entities associated with therapeutic applications has not been previously described or identified. TOS were observed to exist in one of two forms: either as "empty" membrane-bound vesicles or as vesicles containing genetic material. The genetic material was observed to be acquired by the empty vesicles following their entry into tumor cells, as this was often followed by their subsequent emergence with newly acquired contents comprised of genetic material. The TOS were also observed to fuse with one another and appeared to exchange genetic material before dissociating and moving apart. Overall, TOS were observed to be extraordinarily plastic with respect to their ability to fuse with one another to form doublets or even triplet complexes, and to enter tumor cells. This plasticity extends to their genetic contents as well, in that TOS were observed to exchange genetic information with other TOS, and also with cells.

TOS appear to effect their environment by one or more methods of rapid mobility of the TOS, and by methods of exchange or transfer of genetic material. Once produced, the TOS demonstrate (1) the capacity to fuse with other TOS and recombine genetic material before separating, an event that was sometimes associated with cell divisions in duplicate or triplicate. TOS then demonstrate the ability to act on tumor cells by (2) entering tumor cells by membrane fusion, and then inducing rapid morphological changes in the host tumor cell, including but not limited to morphogenesis to the "TOS bags" described above.

The ability of TOS to effect significant tumor changes was demonstrated by their rapid mobility, where TOS were observed to spread rapidly throughout the culture by several means, including the use of extra-cellular matrix (ECM) fibers produced by the tumor cells as pathways from one cell mass to another, and by organizing into tubular masses following their release from tumor cells to form "TAS tubules" as connectors linking various areas of the tumor mass. Thus TOS-tumor cell interactions were associated with tubular extensions of solid tumor masses resulting from the TOS interactions

Pharmaceutical compositions utilizing TOS to affect biological processes may be provided. For example a pharmaceutical composition for the treatment of a mammalian disease may comprise purified TOS having a therapeutic effective activity, combined in a mixture with a pharmaceutically acceptable carrier. A composition is said to be a "pharmaceutically acceptable carrier" if its administration can be tolerated by a recipient patient. For purposes of therapy, compositions comprising TOS and a pharmaceutically acceptable carrier are administered to a patient in a therapeutically effective amount. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995). The choice of a pharmaceutically acceptable carrier, including a physiologically acceptable agent, depends, for example, on the route of administration of the composition by any number of routes known in the art.

In a further example, packaged pharmaceuticals comprising TOS therapeutic anti-cancer activity are provided herein. In one example, the packaged pharmaceutical comprises: (i) an agent comprising a therapeutically effective amount of TOS that is toxic to human tumorigenic cells; and (ii) instructions and/or a label for administration of the agent for the treatment of patients having cancer. In another related example, the packaged pharmaceutical comprises: (i) a therapeutically effective amount of an agent that that interacts with a cellular component that interacts with TOS; and (ii) instructions and/or a label for administration of the agent for the treatment of patients having cancer.

### Therapeutic Indications for TOS

The assessments of the biological functions of TOS indicate it as a therapeutic target in the, treatment of disease in biological organisms, and also as a preventive agent to prevent or halt the development of disease, including but not limited to early stage cancer and metastatic disease. The assessments include but are not limited to discovery of TOS in plant species that display therapeutic anti-cancer activity as demonstrated in pre-clinical assessments, the experimental determination that TOS derived from normal tissue can induce phenotypic reversion and cell death in tumor cells of the same tissue type, and the experimental determination that TOS can induce morphogenetic transformation of target tissue to cells resembling the tissue origins of the transforming TOS particles. Moreover, the diverse biological functions indicate TOS as a tool in the development of novel methodologies for tissue engineering, gene transfer therapeutics and genetic engineering.

TOS derived from normal tissue may be utilized as a therapeutic agent in the treatment of disease. For example, TOS derived from normal tissue has been utilized to induce phenotypic reversion and cell death in tumor cells of the same tissue type. The fact that TOS derived from normal tissue can induce phenotypic reversion and cell death of malignantly transformed cells demonstrates therapeutic value of cell-derived TOS in the detection and treatment of cancer via a novel biological therapeutic approach.

TOS can be used in the treatment of a disease in an animal comprising the steps of providing a source of therapeutic TOS, prepared either by culturing the cells of the animal under conditions that promote TOS formation, or from another cellular source as appropriate based on the nature of the disease condition, followed by TOS purification, then administering the composition to the animal, or to cells derived from the animal, to induce the morphogenetic correction of diseased tissue to produce functional tissue.

### TOS in Genetic Information Transfer and Genetic Engineering

TOS has been demonstrated to enables somatic (horizontal) genetic exchange, cell morphogenesis and intercellular communication as demonstrated herein. TOS exist at least as either as membrane-bound vesicles without genetic material, or as vesicles containing genetic material. Empty vesicles acquire genetic material after entry into cells. TOS may also fuse with one another, exchange genetic material, dissociate and move apart. TOS may fuse to form at least doublet or triplet complexes.

In a further example, a method is disclosed for detecting a genetic abnormality in a patient, comprising, obtaining tissue from a patient, culturing the tissue in vitro to produce TOS from the cells of the patient, isolating the TOS, utilizing the TOS in cellular transformation studies, assessing the phenotypic and genetic results of the TOS cellular transformation studies, and identifying the specific abnormal genes responsible for disease.

In a further example, one or more methods are disclosed utilizing TOS for the introduction of one or more genes into a cell. In one example, TOS may be genetically engineered in vitro to incorporate specific genes for therapeutic transfer. In a further example, TOS may be purified in vitro from cells containing functional genes of interest. Gene transfer to recipient cells may be achieved in culture using the ex vivo methods provided herein. Gene transfer in a recipient may be affected by TOS administered to a recipient via a pharmaceutically acceptable composition wherein TOS comprising one or more genes of interest is combined in a mixture with a pharmaceutically acceptable carrier to comprise a pharmaceutically useful composition for administration to a recipient according to known methods.

In a further example, a method is provided utilizing TOS to transfer one or more genes between cells comprising the steps of (1) selecting one or more cells from a patient or other source having genetic material expressing phenotype characteristics of interest, (2) transferring the genetic material to TOS by incubating the selected cell(s) with a TOS composition, (3) introducing the TOS-genetic material product into a recipient cell, wherein the gene product exhibits the specific phenotype characteristics of the source.

TOS represent an important potential tool for gene therapy. TOS are useful vectors for gene delivery as they efficiently and rapidly interact with cell surfaces, and are readily internalized to elicit rapid morphogenetic effects on the target cell. TOS represent an important tool for gene delivery in that they induce genetic recombination between their genetic contents and the host cell chromosomes to achieve a stable genetic and phenotypic effect in the target cell. TOS may be produced and purified to any desired amount or concentration utilizing the methods provided herein: A method of gene therapy utilizing TOS comprises the steps of providing therapeutic genetic material within TOS by the methods disclosed herein, providing a mixture of the TOS and a pharmaceutically acceptable carrier, and then administering the mixture to a subject in a therapeutically effective amount to deliver the TOS-gene product and produce a phenotypic response.

More particularly, a method of gene therapy for muscular dystrophy utilizing TOS as a gene delivery tool would comprise the isolation of myoblasts from a patient with muscular dystrophy, eliciting TOS--mediated gene transfer of the dystrophin gene to the patient to affect the associated phenotypic conversion of the diseased myoblast to a dystrophin-producing cell. An example of the method comprises transferring the dystrophin genetic material to TOS by incubating dystrophin phenotypic cells with a TOS composition, isolating the TOS-dystrophin product, providing a mixture of the TOS-dystrophin product in a therapeutically effective amount with a pharmaceutically acceptable carrier, administering the TOS-dystrophin mixture to a recipient, wherein the gene product exhibits the specific phenotype characteristics of the source.

In a further example, a method is provided utilizing TOS as a tool for bioengineering of recombinant genotypes based on the facility with which these structures initiate recombination among themselves and with host cells in culture. That TOS enter cells by fusion and exit by fission, or budding, facilitates their use as gene delivery systems.

### Therapeutic Aspects of TOS in Disease and Cancer

There are many potential implications of the involvement of TOS in de novo solid tumor development in furthering our understanding of how cancer develops in its earliest stages to acquire an invasive, metastatic, and genetically unstable phenotype. TOS are novel structural cell-derived entities which have been demonstrated herein to be responsible for multiple aspects of solid tumor development. In another aspect, TOS enables genetic instability in tumors via novel forms of cell fusion and somatic recombination. In another aspect, tumor spread via formation of TOS tubules as conduits facilitates TOS migration, thereby enabling the further spread of TOS, increasing tumor network surface area, increasing cell-to-cell contact in the absence of angiogenesis, and thereby permitting tumor spread without vascularization. In another aspect, TOS enables cell-to-cell contact and tumor cell mass migration to generate solid tumors. In another aspect, TOS has been demonstrated to mediate tumor/stroma micro-environmental interactions associated with proposed epithelial-to-mesenchymal transitions (EMTs), and tumor/stem cell interactions. In another aspect TOS represents a preventive and therapeutic anti-cancer target based on the role in solid tumor formation and spread, analogous to the processes of invasion and metastasis. TOS has been utilized to induce the morphological and physiological transformation of normal tissue into tumors. Thereby demonstrating tumor-derived TOS as an important clinical therapeutic target in the treatment and prevention of cancer. In another aspect, TOS acts as a therapeutic target in that the vascular spread of TOS enables tumor metastases transforming normal tissue at diverse systemic sites remote from the origin of the primary tumor, with the result of metastatic disease. Thus, TOS from cancer cells represent an important therapeutic target in clinical medical treatment of systemic disease.

In a further example, TOS may be utilized to detect cancer in a patient. This test could be done rapidly using previously described TOS purification protocol. The biopsy tissue-derived TOS could then be used in a rapid transformation assay in vitro to assess the ability of the TOS to elicit transformation of standard cultured cells. This test would supplement current histological assessments of tissue malignancy by providing a functional assay of malignant potential in a convenient in vitro system. The method may comprise the steps of obtaining a tissue or other biological sample that will elicit the cancer phenotype from a patient, extraction and isolation of the TOS from the patient sample containing the cancer phenotype material of the source cancer cells, culturing the TOS-cancer phenotype in vitro with standard culture cells capable of being transformed by the TOS-cancer phenotype, and assessing the effect on the culture cells to determine if the phenotype of the source cells is present.

In a further example, a method is provided to identify TOS mediated therapeutic targets in cancer, wherein solid tumors enabled via the TOS mechanism of solid tumor formation and spread, identifies these novel TOS structures as important therapeutic and preventive anti-cancer targets. Based on the documented capacity of TOS to induce the phenotypic reversion of malignant transformed cells in culture, the TOS mediated therapeutic targets may be identified directly by assessing the genetic composition of therapeutic TOS to identify the genes responsible for this therapeutic effect. An embodiment of a method to identify a TOS mediated therapeutic target comprises the steps of culturing purified TOS with recipient cancer cells, assessing the phenotypic response of the cultured cancer cells to determine therapeutic effect of TOS, isolating the DNA from the therapeutic TOS responsible for achieving the therapeutic effect, and analyzing the DNA sequence to identify the gene(s) responsible for mediating therapeutic effect.

TOS represent a potentially useful tool to prevent disease or modulate a disease target due to its capacity to induce morphogenetic tissue transformation. Methods are provided utilizing TOS to treat or prevent disease (e.g., cancer or HD) by modulating the function (e.g., activity or expression) of a target (cellular component) that is identified as provided herein. To illustrate, if a target is identified to promote tumor growth, a TOS therapeutic agent can be used to inhibit or reduce the function (activity or expression) of the target. Alternatively, if a target is identified to inhibit tumor growth, a TOS therapeutic agent can be used to enhance the function (activity or expression) of the target. A TOS therapeutic agent may include, but is not limited to, TOS isolated from non-diseased cells, diseased cells or abnormal cells. A method of preventative treatment of a disease may comprise the steps of purifying TOS from normal functioning tissue, providing a mixture of the TOS and a pharmaceutically acceptable carrier, administering the mixture ex vivo or in vivo to a subject in a therapeutically effective amount, and correcting genetic effects that pose risk for disease development. A method of treatment of a disease may comprise the steps of purifying TOS from normal functioning tissue, providing a mixture of the TOS and a pharmaceutically acceptable carrier, administering the mixture in vivo to a subject in a therapeutically effective amount, and inducing the death of abnormal cells.

In a further example, methods are provided to treat or prevent cancer. TOS derived from normal tissue can be utilized as therapeutic agents in the treatment of cancer based on studies demonstrating that TOS from normal cells are capable of inducing phenotypic reversion of transformed tumor cells in vitro with the result of the induction of cell death. Cancer diseases include, but are not limited to, anal carcinoma, bladder carcinoma, breast carcinoma, cervix carcinoma, chronic lymphocytic leukemia, chronic myelogenous leukemia, endometrial carcinoma, hairy cell leukemia, head and neck carcinoma, lung (small cell) carcinoma, multiple myeloma, non-Hodgkin's lymphoma, follicular lymphoma, ovarian carcinoma, brain tumors, colorectal carcinoma, hepatocellular carcinoma, Kaposi's sarcoma, lung (non-small cell carcinoma), melanoma, pancreatic carcinoma, prostate carcinoma, renal cell carcinoma, and soft tissue sarcoma.

A method of treating or preventing cancer in an individual may comprise administering to the individual a therapeutically effective amount of a TOS composition that is selectively toxic to a tumorigenic cell. The methods comprising the steps as stated above for treatment or prevention of a disease. By "therapeutically effective amount" is meant the amount of the TOS composition administered that is sufficient to elicit the desired therapeutic effect (e.g., the death of a neoplastic cell). It is generally understood that the effective amount will vary according to the weight, sex, age, and medical history of the subject. Other factors which influence the effective amount may include, but are not limited to, the severity of the patient's condition, the disorder being treated, the stability of the TOS composition, and if desired, another type of therapeutic agent being administered with the TOS composition. A larger dose, or a plurality of doses, can be delivered by multiple administrations of the TOS composition. Methods to determine therapeutic efficacy and dosage are known to those skilled in the art For example, therapeutic efficacy may be assessed by standard methods of patient responses to radiation/chemotherapy. In addition, a pretreatment assessment of potential TOS therapeutic effects may be demonstrated on biopsy tumor material cultured with TOS as stated herein.

### Plant Derived TOS and Therapeutics

The discovery of TOS in a primitive plant species implicates this class of cellular structures as a diverse and widespread occurrence in eukaryotic multi-cellular systems with implications for activity in the basic processes of embryogenesis, tissue and organ system development, and tissue repair mechanisms. The novel biological activities of TOS derived from plant species have clinical applications. TOS derived from plant species display therapeutic anticancer activity as demonstrated herein by pre-clinical assessments. More particularly, TOS derived from primitive plant species (fern) demonstrated cytotoxicity. TOS derived from a fern species was found to display therapeutic properties involving the induction of cell death in a human malignant tumor cell line (glioblastoma multiforme). The cell-killing effect of the plant TOS was specific for the malignant tumor cells, a selective cytotoxic response, as comparable treatment of normal cells growing in culture (diploid lung fibroblast W138 cell line) did not produce cytotoxic effects.

The biological functions of plant TOS with therapeutic anti-cancer activity suggest that these structures play an important role in plant defense mechanisms that target diverse cellular systems and adds importantly to the spectrum of TOS activities with potential therapeutic applications. These unique cellular entities may represent a critical cellular component associated with basic processes of metazoan evolution, thereby contributing to a fuller understanding of basic biological processes critical to the structural and functional organization and development of the multi-cellular system. Moreover, the experimental manipulation of TOS may afford tremendous novel opportunities for developing new therapeutic strategies for the treatment of diseased human tissues.

The methods disclosed herein comprise utilizing plant derived TOS to treat or prevent cancer. A method of treating or preventing cancer in an individual may comprise administering to the individual a therapeutically effective amount of a plant derived TOS composition that is selectively toxic to a tumorigenic cell. The methods comprising the steps as stated above for treatment or prevention of a disease.

### TOS and Tissue Engineering

TOS in normal cells enables tissue morphogenesis, development, and embryogenesis. TOS may be utilized as a tool for bioengineering of diverse tissue types based on the observed activities of TOS in the elaboration of complex tissue-like structures and in the induction of major morphogenetic changes in target cells.

TOS may induce morphogenetic transformation of target tissue to cells resembling the tissue origins of the transforming TOS. The genetic and morphological transformations associated with TOS, i.e., producing tissue-specific morphogenesis, has profound implications for the treatment of a broad spectrum of diseases resulting from genetic dysfunction, tissue damage or injury. Moreover, the observed morphogenetic properties of tissue-specific TOS in inducing phenotypic responses in target cells, that are associated with conversion to the tissue type from which TOS originated, demonstrates therapeutic value in the morphogenetic transformation of diseased tissue to a normal phenotype.

TOS enables transfer of genetic material between cells, as discussed above. TOS activity has a profound effect on gene expression consistent with morphogenetic transformation, and the stable transfer of genetic material by means of genomic recombination with recipient cells. The controlled regulatory mechanism directing patterns of gene expression consistent with specific tissue types intrinsic to the mechanism of action of TOS particles demonstrates the corrective genetic remodeling of diseased human tissues of all types using TOS based therapeutics.

Furthermore methods utilizing TOS may be used for tissue engineering or tissue repair, comprising the steps of (1) providing a source of tissue genetic material, (2) culturing the tissue genetic material with purified TOS from normal functioning cells, or other cells chosen for specific properties, to induce TOS to incorporate the cellular genetic material to be utilized as a TOS agent carrying a tissue specific phenotype of the source, (3) purifying and isolating the TOS agent, (4) culturing the TOS agent with target tissue to promote cellular differentiation of the target tissue to the tissue specific phenotype of the TOS agent source, (5) the differentiation being effective for tissue repair. In a further example, a method for producing cells with a tissue-specific phenotype, useful for autogenous tissue repair, comprises the steps of: (1) obtaining tissue from an intended patient; (2) converting the tissue cells into repair and/or replacement cells by culturing with TOS as stated above, the converted cells having effective tissue-specific phenotype activity of the patient.

In a further example, a tissue repair precursor composition, comprising a biocompatible matrix and a dense cell mass growing on the matrix exposed to a culture medium, the dense cell mass being converted to the tissue-specific phenotype exhibiting cell appropriate morphology, the culture medium including at least one TOS agent that promotes tissue-specific phenotype differentiation.

These and other aspects will become evident upon reference to the following detailed description. The invention is set out in the appended claims. The embodiments of the description which do not fall within the scope of said claims are provided for illustrative purposes only and do not form part of the present invention.

### DESCRIPTION OF THE DRAWINGS

Figure 1 is a diagram of an embodiment of TOS formation.
Figure 2 is a photomicrograph (1000x) of purified TOS.
Figure 3 are embodiments of TOS formation: Fig. 3a is a diagram of an embodiment of TOS formation. Figs. 3b - 3d are photomicrographs (1000x) of TOS formation.
Figure 4 are embodiments of TOS cell-to-cell contact: Fig. 4a is a diagram of an embodiment illustrating TOS cell-to-cell contact. Figs. 4b - 4f are photomicrographs (1000x) of cell-to-cell contacts.
Figure 5 is a photomicrograph (1000x) of WI38 normal diploid fibroblasts with no TOS control.
Figure 6 are photomicrographs (1000x) of W138 normal diploid fibroblasts after exposure to TOS.
Figure 7 are photomicrographs (1000x) of normal lung diploid fibroblasts prior to TOS transformation.
Figure 8 are photomicrographs (1000x) of TOS prepared from mouse endothelial cells that contain the GFP (green fluorescent protein) gene.
Figure 9 is a photomicrograph (1000x) of TOS particles stained with acridine orange.
Figure 10 is a diagram of an embodiment of the TOS cell transformation process.
Figure 11 is a photomicrograph (1000x) of TOS transformed lung fibroblasts used for control.
Figure 12 is a photomicrograph (1000x) of transformed TOS lung cells exposed to normal TOS 24 hours.
Figure 13 is a photomicrograph (1000x) of TOS transformed lung fibroblasts exposed to normal lung TOS after ten days.
Figure 14 is a photomicrograph (1000x) of TOS transformed lung fibroblasts exposed to 2X normal TOS 24 hours.
Figure 15 is a photomicrograph (1000x) of TOS transformed lung fibroblasts exposed to 2X normal TOS after 10 days.
Figure 16 is a diagram of an embodiment of cellular reversion by TOS.
Figure 17 is a photomicrograph (1000x) of normal TOS extracted from primitive plant (fern).
Figure 18 are photomicrographs (1000x) of TOS transformed lung fibroblasts exposed to plant TOS at approximately two weeks.
Figure 19 is a photomicrograph (1000x) of normal diploid fibroblasts exposed to plant TOS at approximately two weeks.
Figures 20 are embodiments of TOS tubule formation: Fig. 20a is a diagram of an embodiment illustrating TOS tubules formation; Figs. 20b-g are photomicrographs (1000x) of TOS tubule formation.
Figures 21a - i are photomicrographs (1000x) illustrating TOS bodies in solid tumor architecture.
Figures 22a - c are photomicrographs (1000x) illustrating TOS isolates in culture.
Figure 23 is a sequential series of photomicrographs (1000x) depicting a TOS structure formation.
Figure 24 is video frame showing TOS-mediated movement in NCI-H1299 lung carcinoma cells.
Figure 25 is a video frame showing TOS budding from the surface of a glioblastoma GBM cell.
Figure 26 is a video frame showing TOS-associated tubules and cell extensions in GBM cells.
Figure 27 is a video frame showing TOS attaching to a NCl-H1299 lung carcinoma cell.
Figure 28 is a video frame showing TOS-induced morphological transformation of GBM cell to "TOS bag".
Figure 29 is a video frame showing empty TOS interacting with dense TOS and cell membranes at the border of solid tumor cell.
Figure 30 is a video frame showing TOS-TOS genetic exchange.
Figure 31 is a video frame showing with cell migration and fused TOS with chromosome interactions.
Figure 32 is a video frame showing shows early stage solid tumor formation and dense areas which often serve as initiation sites for TOS formation.
Figure 33 is a video frame showing tubules and extensions associated with TOS activity in GBM solid tumor.
Figure 34 is a video frame showing TOS tubules emerging from cell body serving as conduits for TOS migration.
Figure 35 is a video frame showing individual TOS traveling through a tubule.
Figure 36 is a video frame showing TOS tubule in association with a tumor cell membrane.
Figure 37 is a video frame showing TOS in various stages of budding from a cell membrane.
Figure 38 is a video frame showing TOS-mediated cell-to-cell interactions.
Figure 39 is a video frame showing fused TOS in genetic exchange.
Figure 40 is a video frame showing TOS budding and release.

### DETAILED DESCRIPTION

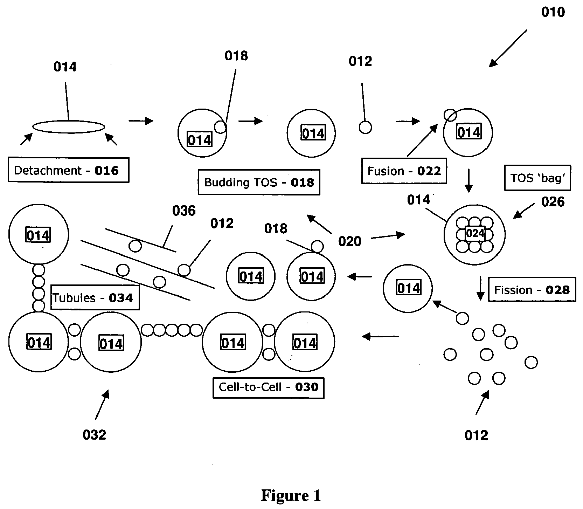

Studies of the earliest stages of solid tumor mass formation in vitro revealed that this process is critically linked to the formation of cell-derived membrane-bound structures, or Tissue Organizing Structures (TOS) as shown in Fig. 1. These previously unidentified structures affect aspects of tumor cell behavior associated with solid tumor development in vitro. The spatio-dynamic data that were obtained from microscopic observations of live tumor imaging and staining for structural assessment indicate that TOS can form as "buds" from the surface membrane of tumor cells, but more commonly appear to form via a fission-like process in which a tumor cell is induced to undergo a rapid morphogenesis to what appears to be a plurality of TOS within the cell, or a "bag of TOS" as discussed herein, that are subsequently released by the rupture or lysis of the tumor cell membrane (Fig. 1).

TOS appear to be membrane-delimited vesicles that frequently contain genetic material based on their staining properties, although empty vesicles that do not contain genetic material have also been observed. Other properties of TOS include rapid mobility and the capacity to fuse with other TOS and recombine genetic material before separating, an event that is sometimes associated with cell divisions in duplicate or triplicate. TOS enter tumor cells by a process that appears to occur by membrane fusion. The TOS then induce rapid morphological changes in the host tumor cell, including morphogenesis to "TOS bags".

TOS were observed to spread rapidly throughout the culture by several means, including the use of extracellular matrix (ECM) fibers produced by the tumor cells as pathways from one cell mass to another. In addition, TOS were capable of organizing into tubular masses following their release from tumor cells to form "TOS tubules" as connectors linking various areas of the tumor mass. These observations indicate that these unique cell-associated structures are highly pleomorphic with respect to their structural parameters and extraordinarily diverse functionally with respect to their ability to interact with each other, with normal or tumor tissue, and to exchange genetic information.

Reference is now directed the Figures which utilize videography, photomicroscopy and diagrams to document the origin of TOS and their varied activities. However, the embodiments of the TOS may be in different forms and these figures should not be construed as limiting the scope of the utility of the TOS as described herein. The figures are illustrious of embodiments and are in accord therewith.

A novel biological entity with-biomedical utility in the form of tissue organizing structures, herein designated "TOS", is described. The TOS were discovered as part of a newly identified process by which cancer cells organize in culture to form three-dimensional solid tumors. The tumor forming process involves these novel cell-derived structures that play a role in orchestrating the process of solid tumor formation and expansion. The studies document that TOS are produced by normal and malignant human cells, and also by cells of murine origin, suggesting that these novel structures are produced by cells of diverse mammalian species. Further studies document the discovery of TOS in species of primitive plants, suggesting that the evolutionary origins of these TOS date to early stages of metazoan evolution. The TOS are implicated in regulating biomedical processes associated with tissue organization and genetic recombination, tissue morphogenesis, malignant transformation, the phenotypic reversion of malignant transformed cells, and the specific induction of cell death in malignant tumor cells.

For a more complete understanding of the TOS and the advantages, reference is now made to the following non-limiting descriptions taken in conjunction with the accompanying examples, protocols and figures.

### Isolation, Purification and Identification of TOS

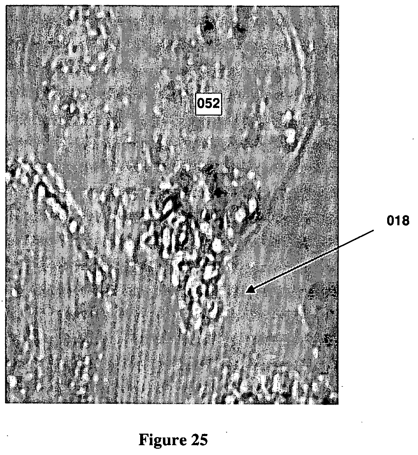

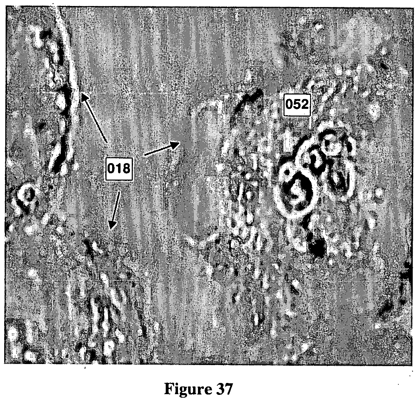

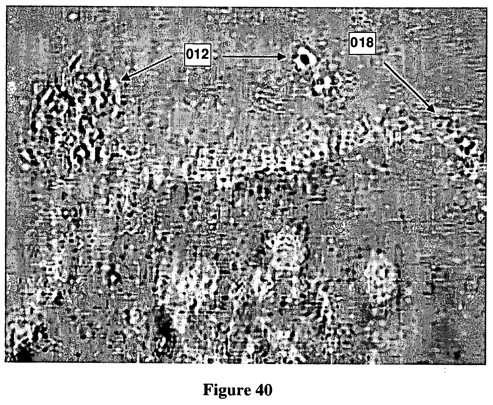

Referring now to the drawings, Fig. 1 is a diagram of an embodiment of TOS formation and tumor growth **010** wherein TOS **012** is induced in genetically altered cells **014,** comparable to hyperplastic cells or emerging carcinoma in situ. As the cells **014** begin to abrogate their normal stromal attachments **016** in the tissue of origin, this initiates cell membrane changes to produce TOS "buds" **018** that induce the formation and release of TOS **012,** a process that may be driven by a positive feedback loop mechanism **020,** as TOS **012** give rise to more TOS **012** by subsequent 'n' rounds of fusion **022** with additional tumor cells **014** that also may trigger autonomously driven intracellular TOS replication **024** and reproduction of a cell into a plurality of TOS in the cell, or a TOS "bag" **026** as utilized herein, and then with rapid cell fission **028** TOS bodies **012** are released. Free TOS bodies **012** mediate cell-to-cell attachments **030** enabling tumor **010** growth. In an additional embodiment of tumor growth **032,** free TOS bodies **012** aggregate to form TOS tubules **034** utilizing cellular ECM fibers **036.** These tubules **034** then act as conduits and direct connectors to enable tumor **0320** growth. TOS **012** also appear to stimulate cell division in tumor cells **014** and somatic genetic recombinations that may contribute to genetic instability and morphogenetic changes occurring in tumors cells **014** with which they are associated. The formation of TOS tubules **034,** their utilization of ECM components **036,** their rapid mobility all taken together may orchestrate many of the dramatic cell movements that characterize the abnormal tissue organizations comprising solid tumor **010** and **032** structural components. Moreover, the TOS tubules **034** generate a circuitry of tumor cell origin that permits extensive tumor **032** spread and communication in the absence of angiogenesis. The activities of these unusual TOS bodies **012** account for many of the abnormal behaviors characteristic of solid tumor malignancy. In addition, Fig. 26, a frame from live cell videography, shows TOS-associated tubules **034** and cell extensions of GBM cells **052.** Fig. 37, another frame from unstained live cell videography, shows TOS in various stages of budding **018** from a GBM cell **052** membrane. Fig. 40 is a frame from unstained live cell videography showing TOS budding and release from MDA-MB-231 breast carcinoma cell.

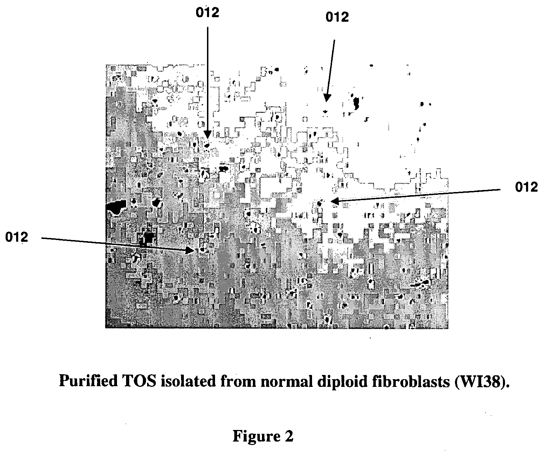

Fig. 2 is a photomicrograph (1000x) of an embodiment of TOS **012** isolated from normal diploid fibroblasts (WI38), the method of isolation comprising the steps of;
a. culturing W138 cells overnight in serum-free culture medium to stimulate the production of TOS in the cultured cells,
b. removing the culture media,
c. washing the cells with a composition of phosphate buffered saline,
d. treating the cells with a composition of trypsin for 30 minutes to facilitate cell accumulation of TOS,
e. adding 10% fetal bovine serum (FBS) to the trypsanized cells to stimulate release of TOS,
f. releasing TOS from the trypsanized cells by incubating overnight at 37 degrees centigrade wherein the released TOS bind tightly to the bottom of the culture dish,
g. removing the cells from the culture dish to isolate the bound TOS.

An embodiment of producing a purified composition of TOS **012** comprises the steps of,
a. culturing W138 cells overnight in serum-free culture medium to stimulate the production of TOS in the cultured cells,
b. removing the culture media,
c. washing the cells with phosphate buffered saline,
d. treating the cells with trypsin for 30 minutes to facilitate cell accumulation of TOS,
e. adding 10% fetal bovine serum (FBS) to the trypsanized cells to stimulate release of TOS,
f. releasing TOS from the trypsanized cells by incubating overnight at 37 degrees centigrade wherein the released TOS bind tightly to the bottom of the culture dish,
g. removing the cells from the culture dish to isolate the bound TOS,
h. removing the bound TOS from the culture dish by physical or other acceptable means to substantially maintain TOS activity, such as a cell scraper, and
i. resuspending the TOS in an amount of acceptable diluent to maintain TOS activity, such as culture medium, to provide a purified TOS composition.

The product is one hundred percent (100%) pure TOS as cells from which they were derived are destroyed by selective trypsinization. No other steps are required for purification. Other embodiments for TOS isolation and purification comprise high speed ultracentrifugation.

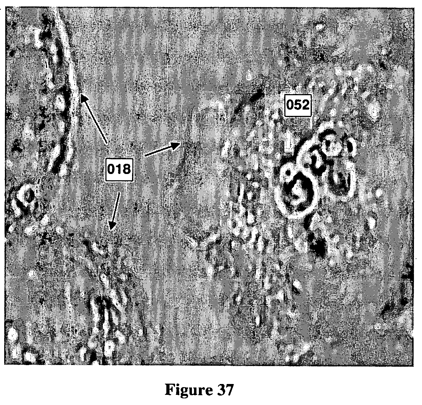

An embodiment for the study of tumor formation and isolation of TOS in vitro comprises utilizing tumor cells derived from diverse human cancer cell lines, including but not limited to cancers of the colon, lung, brain and breast, by plating the tumor cells following trypsinization on culture dishes coated with 1% agarose to block cell-to-substrate attachment in order to promote cell-to-cell interactions required for solid tumor formation. Videography or photomicroscopy imaging in real time was conducted of the live cell cultures, from the time of plating continuously for 4-5 hours post-plating and intermittently over a period of approximately one month. This procedure was utilized to record the overall chronology of early stage solid tumor development and to explore visually the behavior of individual cells as they begin the process of spontaneous associations that culminate in the formation of microscopic solid tumors in vitro. In addition, photomicroscopy and videography documentation of the tumor cell behavior was conducted to identify the TOS. For example, Fig. 37 is a frame from unstained live cell videography showing TOS in various stages of budding **018** from a GBM cell 052 membrane. Fig. 38 is a frame from unstained live cell videography showing TOS-mediated cell-to-cell interactions **031** (MDA-MB-231 breast carcinoma cells).

An additional embodiment of a method for isolating TOS comprises culturing cells in the absence of serum in pyruvate supplemented medium that generated conditions not conducive to sustaining cell viability while facilitating the culture and isolation of cell-free extracts of TOS for further characterization.

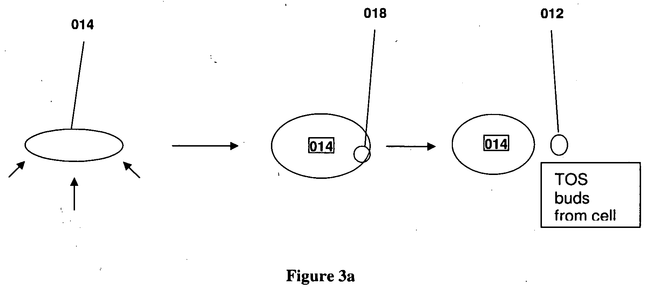

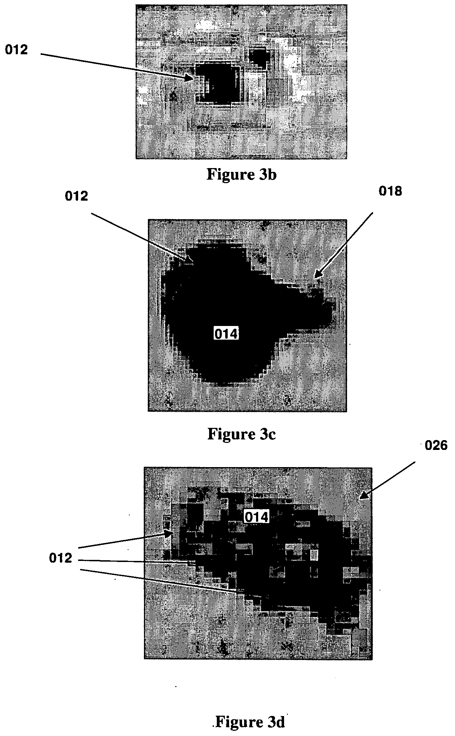

An embodiment of a method for identification of TOS comprises staining fresh tumor tissue with either hematoxylin/eosin, or periodic acid Schiff reagent/hematoxylin by known methods. Visualizing of the TOS by microscopy is employed with a record made by known photographic techniques. TOS isolated and stained according to the method described herein is identified in the photomicrograph of Fig. 3 at 1000x magnification having an approximate diameter of 2 microns in diameter.

TOS may be isolated from cells growing in culture by microscopic visualization of cells immediately following substrate detachment, an event that triggers TOS formation and budding from the cell surface. TOS may be visualized at high magnification (1000X) in live unstained cell preparations in culture and by staining (Figs. 2, 3, 17, 22, 29, 30, 31, 35, 36 and 40). TOS morphology is spherical and the diameter is approximately 2 microns. Hematoxylin/eosin (H & E) staining reveals a densely staining interior of many, but not all, TOS particles, suggestive of nucleic acid content. Periodic acid/Schiff (PAS) staining of TOS particles stains the exterior surface and many extensions/tubules associated with TOS indicating the presence of lipid or lipids. The exterior surface is membrane like in its properties (budding, fusion) and its lipid-staining biochemical properties. The presence of nucleic acid in the interior of the TOS vesicles was confirmed using acridine orange staining and fluorescence microscopy, which indicated the presence of double-stranded nucleic acid in these particles. Rhodamine 1,2,3-staining produced a pronounced fluorescence indicative of energy production in these vesicles. Protein does not appear to be a major component of TOS particles as they are insensitive to trypsinization.

### TOS Assessment as Therapeutic Cancer Agent or Target

TOS possess unique properties associated with tissue organization and genetic recombination and have clinical utility in tissue and genetic engineering as well as in cancer prevention and therapeutic use and targeting. The studies of the earliest stages of solid tumor mass formation in vitro revealed that this process is critically linked to the formation of TOS bodies. These previously unidentified structures appear to play a central role in many aspects of tumor cell behavior associated with solid tumor development in vitro.

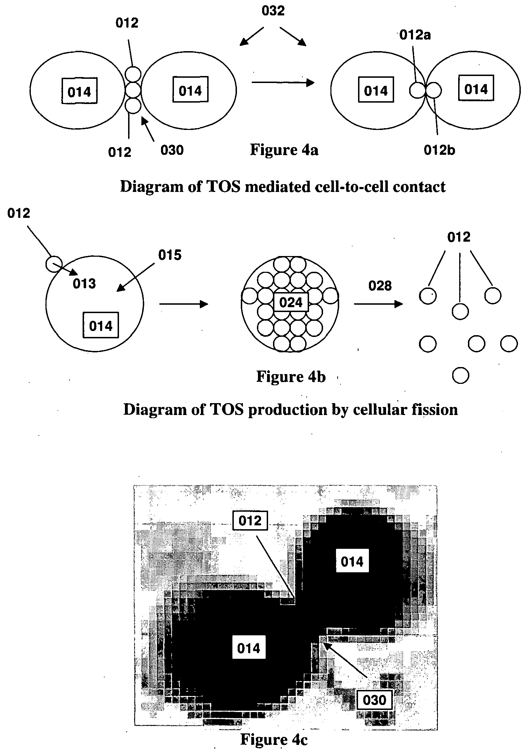

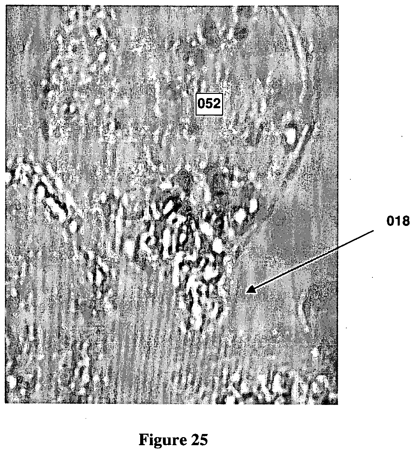

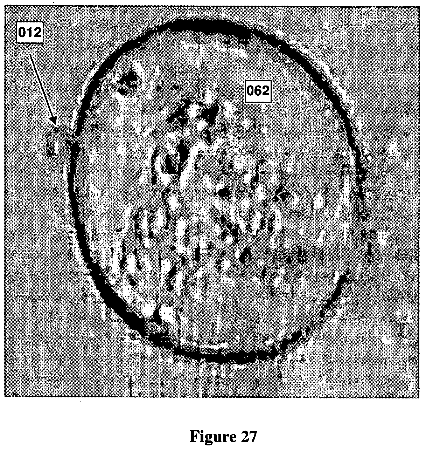

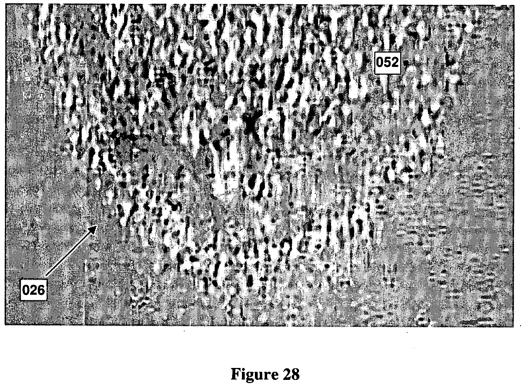

In the embodiment of Fig. 3a, a model of TOS production by budding is presented wherein a TOS particle **012** "buds" **018** from a tumor cell **014.** Fig. 3b is a photomicrograph (1000x) of an individual TOS body **012** isolated from hematoxylin/eosin stained, glioblastoma multiforme DBTRG.05MG cells, having an approximate size of 2 microns. A single particle is shown with a densely stained central area. In Fig. 3c, the spatio-dynamic data that was obtained from microscopic observations of live tumor imaging and H & E staining for structural assessment indicated the TOS bodies **012** may form as "buds" **018** on the surface membrane of tumor cells **014** and are then activated by substrate/stromal detachment **016.** The image shows the morphogenesis of tumor cell (GBM) to TOS - producing cell. Note the interior of densely staining TOS aggregates. Fig. 3d is a photomicrograph (1000x) of a tumor cell **014** showing TOS bodies **012** formed via a fission-like process (also see Fig. 4a) in which a tumor cell **014** is induced to undergo a rapid morphogenesis and then lyse to release a plurality of TOS **012.** The H & E stained cell preparation shows complete conversion of the tumor cell (GBM) to a "TOS bag"- a cell consisting of emergent TOS particles to be released by cell fission. The densely stained interior particles are associated with TOS formation. In addition, Fig. 25, a frame from live cell videography, shows TOS budding **018** from the surface of glioblastoma GBM cells **052.** Fig. 27, another frame from live cell videography, shows TOS **012** attaching to NCI-H1299 lung carcinoma cells **062.** Fig. 28, another frame from live cell videography, shows TOS-induced morphological transformation of GBM cell **052** to "TOS bag" **026.**

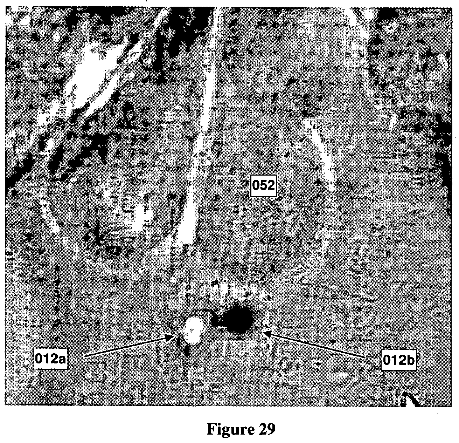

TOS **012** in Fig. 3 appear to be membrane-delimited vesicles that frequently contain genetic material based on their staining properties, although empty vesicles that do not contain genetic material have also been observed. Fig. 29, a frame from live cell videography shows empty TOS **012a** (light) interacting with dense TOS **012b** (dark) and cell membranes at the border of solid tumor GBM cells **052.** Other properties of TOS include rapid mobility and the capacity to fuse with other TOS **012** and recombine genetic material before separating, an event that is sometimes associated with cell divisions in duplicate or triplicate. Alternatively, TOS **012** may enter tumor cells **014** by a process that appears to occur by membrane fusion **022** and then induce rapid morphological changes in the host tumor cell **014,** including morphogenesis to "TOS bags", which is a cell **014** containing a plurality of TOS **012** prior to lysing and release.

TOS **012** were observed to spread rapidly throughout the culture by several means, including the use of extra-cellular matrix (ECM) fibers **036** produced by the tumor cells **014** as pathways from one cell mass **032** to another. In addition, TOS **012** were capable of organizing into tubular masses following their release from tumor cells to form TOS tubules **034** as connectors linking various areas of the tumor mass **032,** as shown in Figs. 1.

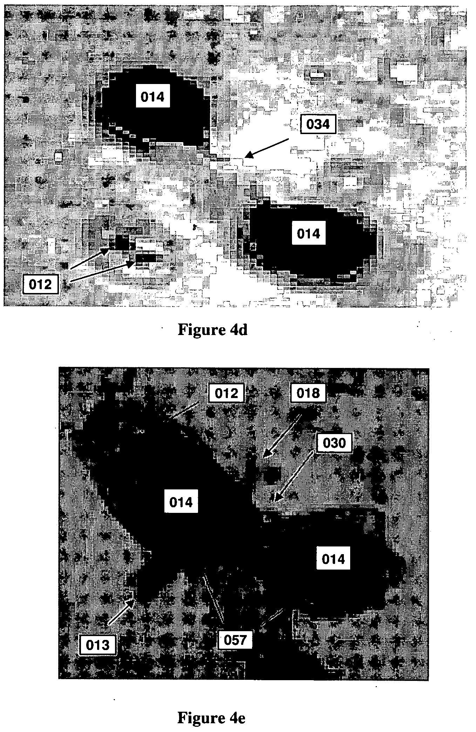

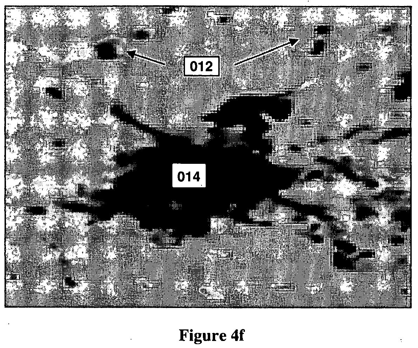

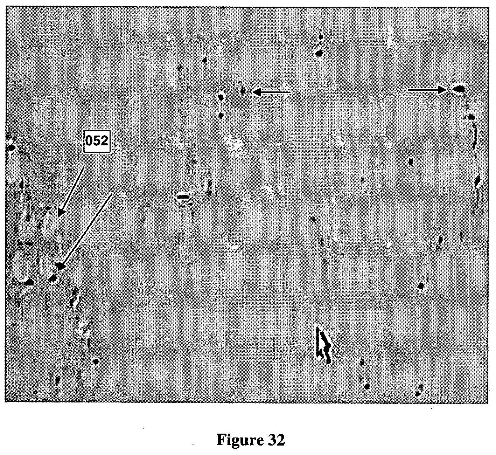

Figs. 4a and 4b are embodiments of two diagrams depicting TOS mediated cell contact and TOS produced by cellular fission. In Fig. 4a, solid tumor formation **032** is indicated generally showing that TOS bodies **012** and/or tubules **034** formed from TOS **012** mediate cell **014** to cell **014** contact **030** to produce cells conjoined by TOS bodies **012a** and **012b.** This process is repeated thus allowing TOS **012** to act as the mediator for tumor **032** growth. Fig. 4b illustrates generally TOS **012** production by cellular fission **028,** where cell **014** is contacted by TOS body **012** which infuses TOS material **013** into the cell **014**cytoplasm **015,** TOS bodies **012** then replicate **024** within the cell **014** and are released with cell fission or lysis **028.** Figs. 4c is a photomicrograph (1000x) of NCl-HI299 lung carcinoma, hematoxylin/eosin stained cells **014,** at day one of solid tumor **032** formation showing TOS **012** mediated cell fusion in cell-to-cell contacts **030** associated with TOS **012 in** accord with the illustration above. Fig. 4d is a photomicrograph (1000x) of H & E stained GBM tumor cells **014,** illustrating that TOS **012** mediated cell fusion may progress to elaborate tubular structures, TOS . tubules **034,** between cells. Fused TOS particles **012** with densely staining interiors interacting may indicate genetic recombination. Figs. 4e is a photomicrograph (1000x) of DBTRG.05MG glioblastoma multiforme, periodic acid/Schiff reagent stained cells, showing cell-to-cell contact **030.** The fused tumor cells **014** display internal TOS **012** formation, individual TOS budding **018,** and a larger aggregate **013** budding from cell **014.** Note the enlarged nuclei **057.** Fig. 4f is a photomicrograph (1000x) of an H & E stained GBM tumor cell **014** showing fission of the TOS filled cell and release of TOS particles **012.** The net result of these TOS activities appeared to be directly associated with cell-to-cell associations and the formation of intercellular structures to facilitate the association and spread of tumor masses within the culture vessel. In Fig. 32, a frame from unstained live cell videography, early stage solid tumor formation of GBM cells **052** in culture is shown. Note the arrows indicating dense areas concentrated at sites of cell polarization and migration. These often serve as initiation sites for TOS formation.

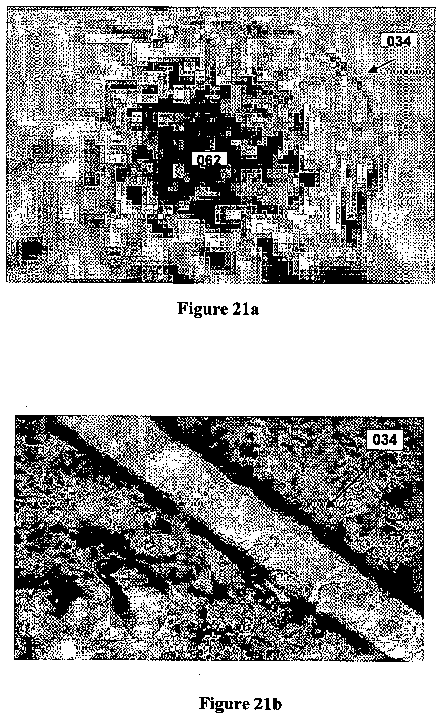

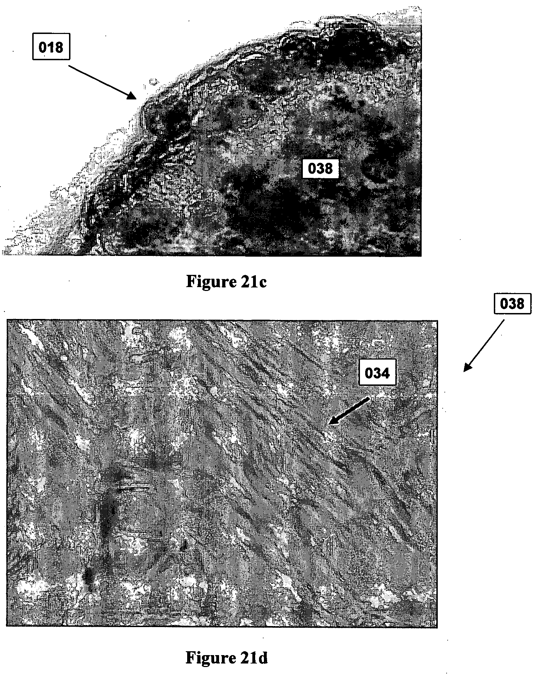

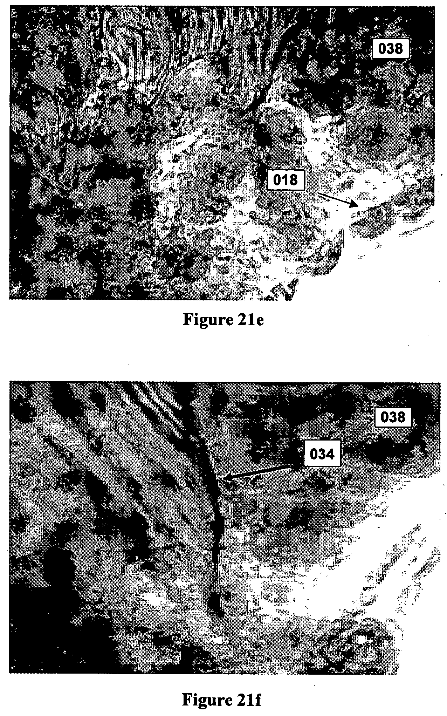

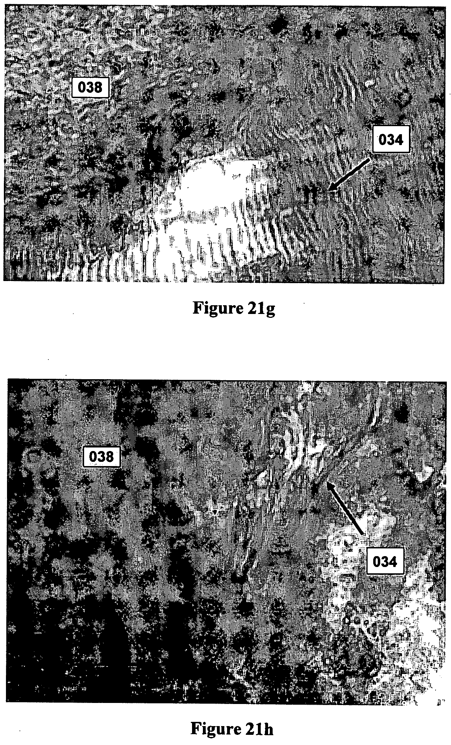

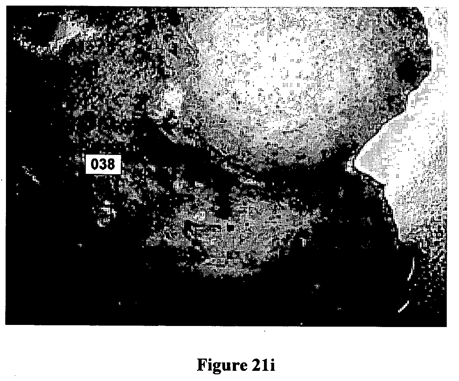

Based on the live microscopy studies of the spatio-temporal behavior of tumor cells at early stages of solid tumor formation in vitro, it appears that TOS are plasma membrane delimited cell-derived structures that conduct a central role in orchestrating the movements and cell-to-cell interactions of tumor cells essential to the formation of microscopic solid tumors, and is the presumable initiation complex fundamental to the establishment of solid tumor malignancies. In Fig. 21, the observed TOS-tumor cell interactions and tubular extensions of solid tumor masses resulting from the TOS interactions suggest that these novel structures may play a critical role in processes associated with tumor spread and metastasis, both fundamental components of the malignant phenotype.

As shown in Figs. 3 and 4, TOS exert a dramatic capacity to effect structural and functional alterations in tumors with which they associate almost immediately upon entry into the tumor cell, a process that appears to occur by cell fusion. This effect is clearly manifest in changes in the appearance of the cell surface, the movements of the cell induced by this association and also by the apparent interaction of these vesicles with the genetic material of the tumor cell, both as a mechanism by which they may acquire their genetic contents and also by altering the genetic contents of the tumor cell. These TOS-tumor cell associations also appear to serve as a stimulus for tumor cell fission to form "TOS bags" that subsequently release with cell lysis a plurality of TOS bodies that spread throughout at least the tumor area. This fission process may represent a novel form of autonomous replication process.

### Malignant Cell Induction or Transformation by TOS

Tissue organizing structures (TOS) derived from human malignant tumor cells are capable of inducing the morphological and biological phenotypic transformation of normal diploid fibroblast cells to a highly aggressive, malignant transformed cell line that was established as a stable immortalized cell line in vitro for several months.

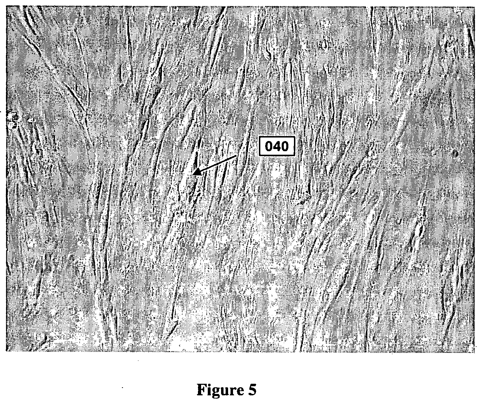

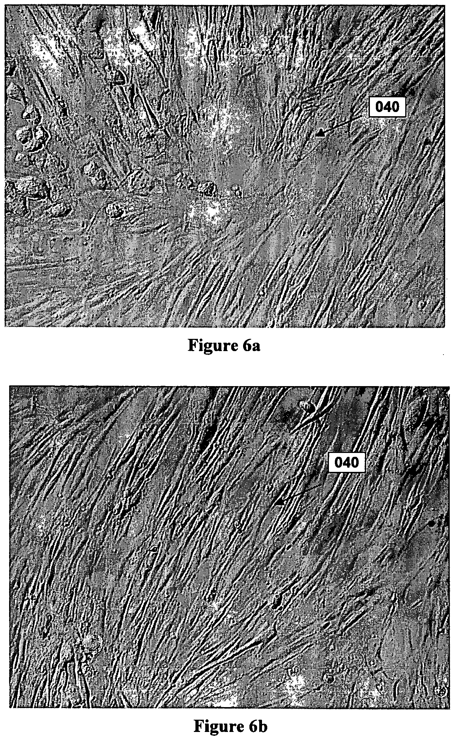

Fig. 5 is a photomicrograph (1000x) of a live cell preparation of normal W138 lung diploid fibroblasts **040** prior to exposure of TOS control. Figs. 6a and 6b are photomicrographs (1000x) of live cell preparations of normal diploid lung fibroblasts (WI38) **040** after exposure to TOS control **044** (not shown) derived from W138 normal diploid fibroblasts **040.** The figures demonstrate untreated control versus cells treated with normal TOS, which produced no demonstrable morphological effect.

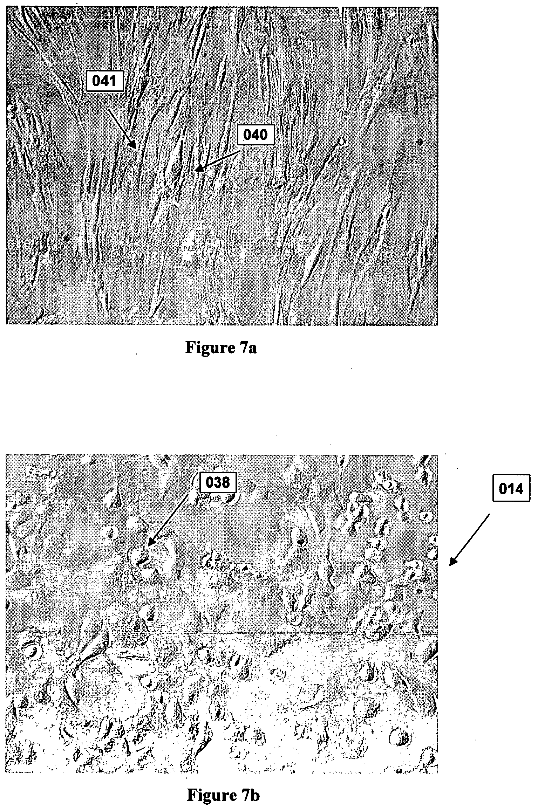

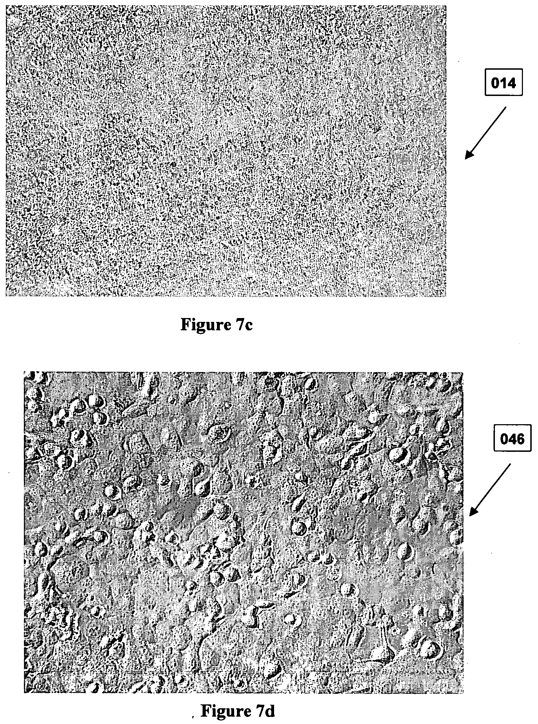

Figs. 7a - 7d are photomicrographs (1000x) of unstained live cell preparations of normal diploid lung fibroblast (WI38) cells **040** in monolayer culture. Fig. 7a shows normal lung diploid fibroblasts **040** prior to TOS **042** exposure and transformation. The elongated fibroblastic cell extension **041** is a characteristic morphology of this cell line. In Fig. 7b the normal cells **040** have been exposed to TOS **042** (not shown) derived from NCl-HI299 non-small cell lung carcinoma. Note the dramatic change in normal cell **040** morphology where the cells **040** have lost their characteristic fibroblastic extensions **041** thus showing malignant transformation of the normal lung fibroblasts **040.** Within several days the **040** cells began to assume a rounded-up, spheroid **038** morphology and developed biological properties of malignant transformed cells **014.** Fig. 7c is an unstained live cell photograph showing the TOS transformed fibroblasts **014** after 10 days resulting in a confluent monolayer of TOS-transformed lung fibroblasts. Fig. 7d is a photomicrograph of live cells showing the TOS transformed cell line **046** after several weeks in culture. The TOS **042** mediated morphogenetic transformation generated a stable phenotypic alteration. The morphogenetic properties of these cells **046** were distinct from the normal diploid fibroblasts **040** from which they were derived. They are similar in appearance, but display aggressive growth properties, unlike the finite normal cell line **040** from which they originated, similar to the lung carcinoma cells used to prepare TOS **042** for the transformation.

The methodology to transform the normal cells into malignant cells having the phenotype of the source malignant cells involved the application of purified TOS **042** prepared from the NCl-HI299 non-small cell lung carcinoma malignant cells to a monolayer culture of normal lung fibroblasts **040,** comprising the steps of:
a. isolating and purifying TOS from NCl-H1299 lung carcinoma cell using the procedure described above,
b. suspending TOS in phosphate buffered saline solution isolated from approximately 2 X 10^5 cells,
c. adding the TOS suspension to a culture dish containing a monolayer of normal WI38 lung cells (approximately 1 X 10^5 cells) growing in culture medium,
d. incubating the TOS-treated cells at 37C in 5%CO2, and
e. producing TOS transformed malignant cells comprising the phenotype of the source malignant cells.

The TOS **042** and cell **040** interactions resulted in the morphological transformation of the normal lung fibroblasts **040** to cells **046** that distinctly resembled the lung carcinoma cells from which the TOS **042** were derived. Morphological transformation occurred over a period of several days to one week and produced a stable cell line **046** displaying many properties of transformed cells **014,** including: immortalized growth, rapid mitosis, anchorage independence and ability to form solid tumors in vitro. These malignantly transformed cell lines **046** were maintained in culture for a period of more than three months without any loss of proliferative capacity, thus behaving as a continuous cell line, distinguishing them from the finite fixed lifespan cell line **040** from which they were derived.

These cell lines **046** have been maintained as a genetically stable novel cell line **046** in vitro capable of anchorage-independent growth and these cells form solid tumor spheroids **038.** In contrast, the pre-transformed normal W138 cells **040** require substrate attachment characteristic of anchorage-dependent growth and viability, and are incapable of generating solid tumor spheroids **038,** a characteristic of malignant cells. Thus the TOS-transformed cell line **046** displays widely divergent properties distinguishing these transformed cells **014** from the normal lung fibroblasts **040** from which they were derived. Moreover, the TOS-transformed cells **046** display growth properties in vitro associated with a higher proliferative rate than the lung carcinoma cells from which the TOS were derived, consistent with a highly malignant transformed state.

This data suggests a novel mechanism of malignant transformation associated with TOS activity, and has increased the scope of functional activities associated with these biological entities. This mechanism has important potential clinical applications defining TOS as a therapeutic target based on the observed transforming behaviors of tumor-derived TOS.

The observed phenotypic transformation suggests that TOS are capable of transferring genetic material to host cells with which they are associated in a stable fashion which may be associated with genomic integration of exogenous TOS-associated DNA. The DNA transfer function of TOS is further indicated by additional studies demonstrating that TOS derived from green fluorescent protein(GFP)-transformed mouse endothelial cells are cable of inducing the phenotypic transformation of human glioblastoma multiforme (GBM) tumor cells such that they acquire a GFP+ fluorescent phenotype following exposure to TOS derived from GFP+ cells.

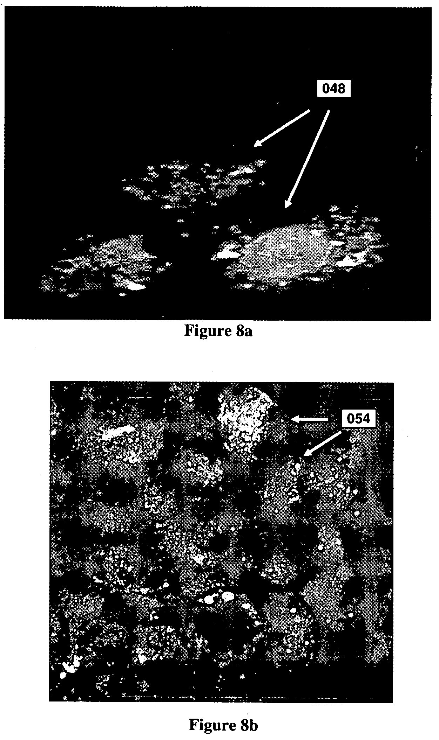

In Figs. 8a and 8b (Confocal laser fluorescent microscopy I000x), TOS **048** prepared from mouse endothelial cells that contain the GFP (green fluorescent protein) gene **050** transformed human glioblastoma multiforme cells (GBM) **052** to a GFP+ fluorescent phenotype cell **054** by the method comprising the steps of,
a. isolating and purifying TOS from mouse endothelia cells that contain the GFP gene using the procedure described above,
b. suspending the TOS in phosphate buffered saline solution,
c. adding the TOS suspension to a culture dish containing a monolayer of human glioblastoma multiforme cells (GBM) growing in culture medium,
d. incubating the TOS-treated cells at 37C in 5%CO2, and
e. producing TOS transformed cells comprising the GFP+ phenotype of the source cells.

Additional studies documented the presence of DNA **056** in TOS by acridine orange staining and fluorescent photomicroscopy by methods known in the art, comprising
a. isolating and purifying TOS from cells in culture by the methods described herein,
b. staining the TOS particles with acridine orange fluorescent stain according to standard established procedures,
c. and examining the stained TOS particles by fluorescent microscopy.

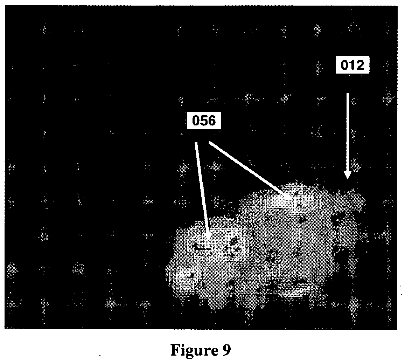

In Fig. 9, TOS particles **012** stained with acridine orange indicate the presence of double-stranded DNA **056** by confocal fluorescent laser microscopy (1000x).

These TOS-transformation studies suggest that the source of the DNA 056 component of the TOS **012** is the host cell genomic DNA as the phenotypic properties of TOS-transformed cells **014** vary depending on the cell source of the TOS **012.** These studies further suggest that TOS **012** are capable of engaging in genetic recombination between DNA **056** sequences carried in the TOS **012** and host cell genomic DNA. The recombinational properties of TOS **012** may comprise the basis of their ability to induce the morphogenetic transformation of cells with which they interact. These recombinational properties have profound applications for novel gene therapy approaches to the treatment of many diverse human diseases.

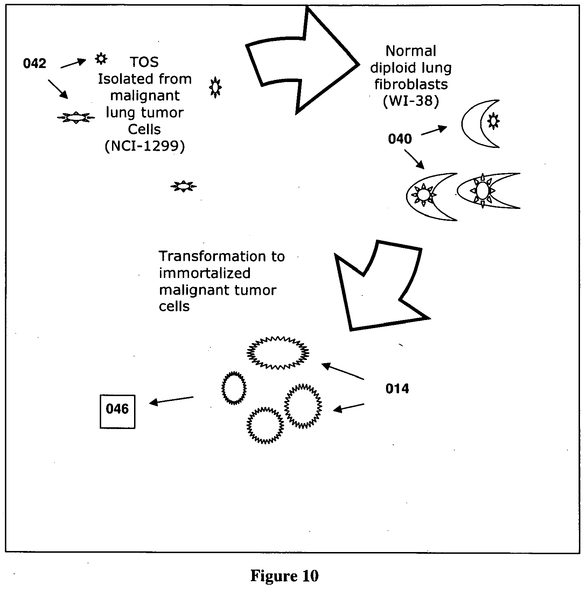

Fig. 10 is a diagram of an embodiment of a method of a TOS cellular transformation process. TOS **012** extracted and purified from malignant lung carcinoma cells **042** are added to cultures of normal diploid fibroblasts **040.** Within several days, the normal cells **040** begin to assume the properties of transformed malignant cells **014** and can be established as a permanent cell line **046.**

### Malignant Cell Reversion by TOS

Tissue organizing structures (TOS) **044** derived from normal diploid fibroblasts **040** are capable of inducing the morphological and biological phenotypic reversion of the malignant cell line **046** established by TOS transformation, ultimately inducing transformed malignant cell death.

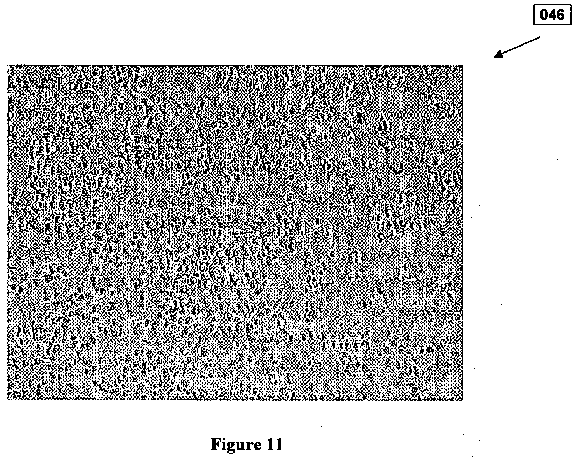

Fig. 11 is a photomicrograph (1000x) of an unstained live cell preparation of TOS transformed lung fibroblasts **046** as the control wherein no normal TOS has yet been added, and thus acts as a negative control.

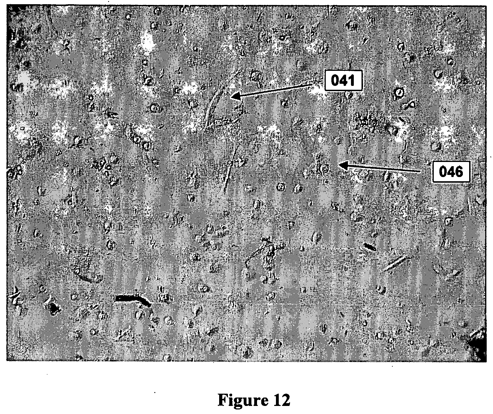

Fig. 12 is a photomicrograph (1000x) of an unstained live cell culture of TOS transformed tumorigenic lung cells **046** exposed to normal TOS **044** (not shown) after 24 hours. TOS **044** induce a partial phenotypic reversion of the lung carcinoma cells **046** as cell extensions **041** characteristic of normal fibroblasts **040** are identified. Thus, the photo shows evidence of morphogenetic reversion of TOS-transformed cells to a more fibroblastic appearance and evidence of cytotoxicity.

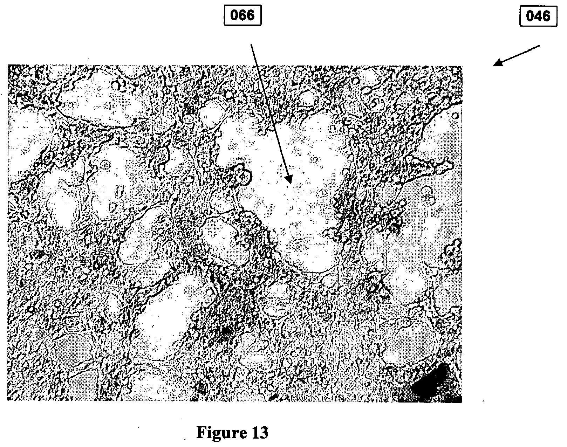

Fig. 13 is a photomicrograph (1000x) of an unstained live cell culture of TOS transformed lung fibroblasts **046** exposed to normal lung TOS **044** after ten days. At ten days, the TOS-treated malignant cells are growing poorly with open areas appearing as syncytial plaques **066.** Thus, the photo shows evidence of massive cell death of transformed cells, suggesting that TOS from normal cells may have a therapeutic destructive effect on abnormal malignantly transformed cells in culture.

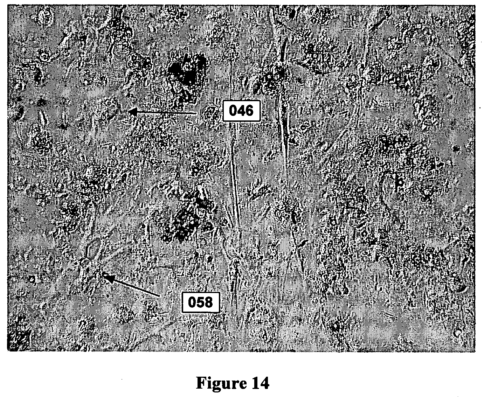

Fig. 14 is a photomicrograph (1000x) of an unstained live cell culture of TOS transformed lung fibroblasts **046** exposed to 2X normal TOS **044** 24 hours. Phenotypic cell reversion **058** is concentration dependent as pronounced morphological effects are observed when 2X purified TOS is applied to malignant cells **046** growing in culture. **058** indicates a morphologically transformed cell. Thus, the photo shows concentration-dependent effects of therapeutic TOS activities

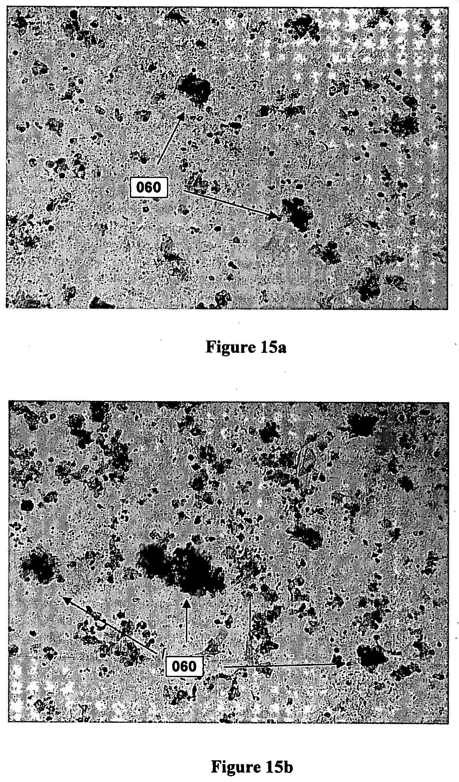

Fig. 15 is a photomicrograph (1000x) of an unstained live cell preparation of TOS transformed lung fibroblasts **046** exposed to 2X normal TOS **044** after ten days. The increased concentration has produced clusters of dead cells **060.** Compare to the cell death in Fig. 13.

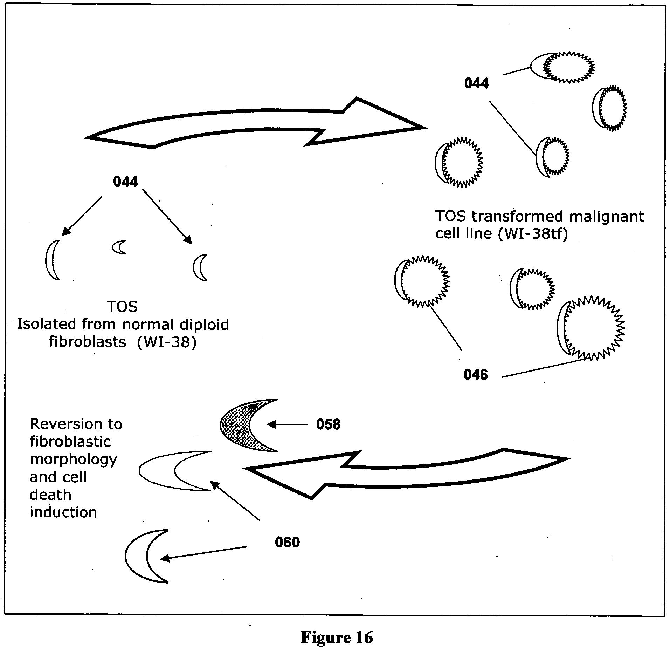

Fig. 16 is a diagram of an embodiment of cellular reversion by TOS. TOS **044** derived from the normal lung fibroblast cell line (WI38) **040** were observed to induce the phenotypic reversion **058** of previously TOS transformed lung cells **046** such that they assumed a normal fibroblastic phenotype **040** which preceded the induction of cell death **060** in the TOS-transformed cells **046.** Procedurally, TOS **044** extracted and purified from normal WI-38 human diploid fibroblasts **040** were added to cultures of TOS-transformed malignant cell line **046.** Within several days phenotypic reversion **058** and cell death **060** occurred in the transformed cell line **046.** The rate of phenotypic reversion **058** and degree of cell death induction **060** were correlated with the amount of purified TOS **044** added to the cultured transformed cells **046.** Although quantitative methods of TOS assessment are currently still in development, it was estimated that TOS **044** purified from approximately 1X 10>4 cells **040** were capable of inducing the complete cell death **060** of 1X 10>6 cells **046** per mL in culture. This observed phenotypic reversion **058** of a malignantly transformed cell line **046** by TOS **044** derived from normal cells **040** suggests that TOS **044** may incorporate functions that have significant potential therapeutic value. In addition, it was observed that the TOS **044** derived from normal lung cells **040** induced the death **060** of the phenotypically reverted transformed cells **046,** while displaying no discernible morphogenetic or toxic effects when combined with the normal lung cells **040** from which they were isolated. These data provide further evidence that TOS **012** derived from different cell and tissue types contain unique DNA **056** components specific to their tissue of origin and are capable of effecting morphogenetic alterations of unrelated tissues, including the reversion **058** of a disease-associated phenotype.

TOS derived from normal tissue can be utilized as therapeutic agents in the treatment of cancer based on studies demonstrating that TOS from normal cells are capable of inducing phenotypic reversion of transformed tumor cells in vitro associated with the induction of cell death.

### TOS Isolated from Primitive Plant Species

TOS derived from plant tissue can be utilized as therapeutic agents in the treatment of cancer based on their ability to elicit selective cytotoxic responses in human malignancies.

In addition, TOS were identified and purified from a species of fern, indicating that these novel cell entities are ubiquitously present in widely divergent phyla, suggesting a common evolutionary origin that may be associated with the beginnings of the metazoan system. This notion is consistent with the apparent tissue organizing regulatory activities of these biological entities which may implicate these sub-cellular structures in embryogenic and developmental pathways essential to the formation of the multi-cellular system.

An embodiment of plant TOS extraction and purification procedure comprises: Plant material (leaves and stems) are washed in alcohol, then rinsed with sterile water. Cell culture media containing 10% FBS is added to the cleaned plant material. The mixture is then homogenized for approximately 3 minutes. The homogenate is then cultured at 37C for 24-72 hours. The media/homogenate is removed leaving the plant TOS adhered to the culture flask. Plant TOS are scraped from the flask with a cell scraper in pure form in the absence of plant cells or other materials.

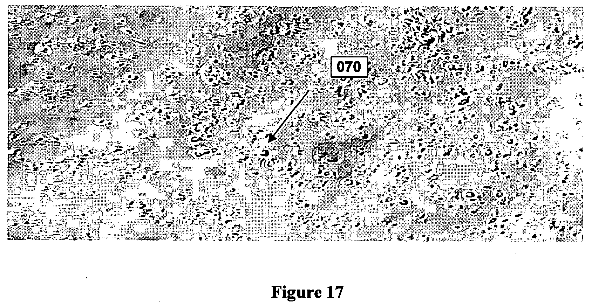

Fig. 17 is a live cell photomicrograph (1000x) of normal TOS **070** extracted from primitive plant (fern).

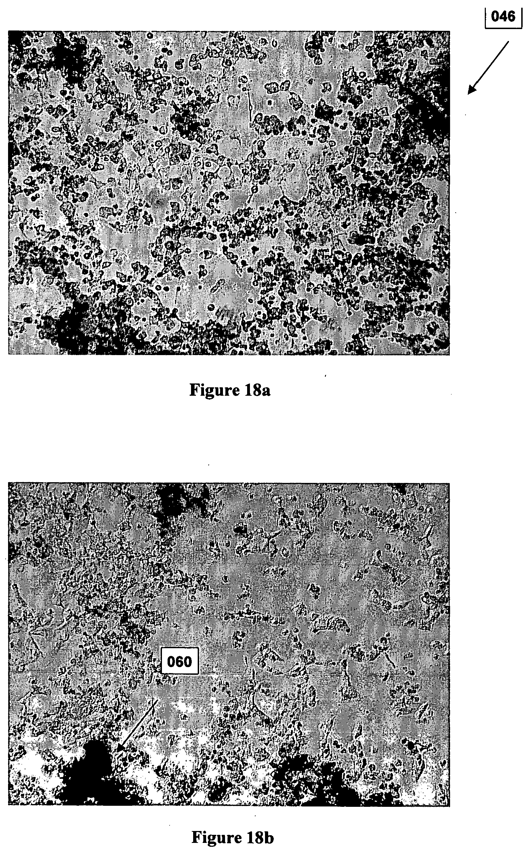

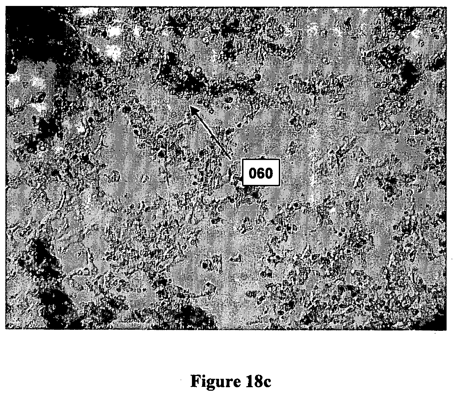

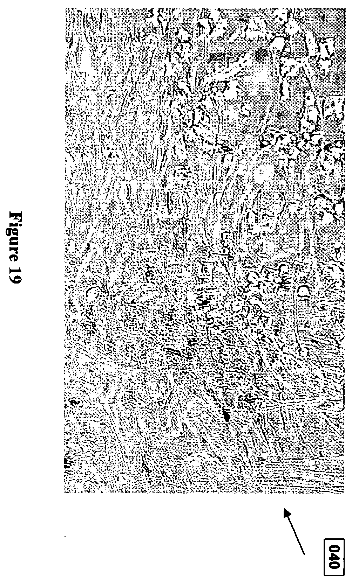

In Figs. 18 and 19, TOS isolated from the fern species were found to display important cytotoxic activities specifically targeting malignant cancer cells while sparing normal cells in culture. Figs. 18a - 18c are photomicrographs (1000x) of unstained live cell preparations of TOS transformed lung fibroblasts **046** exposed to plant TOS **070** (not shown) at approximately two weeks. Note the clusters of dead cells **060** as in Fig. 15. Compare the result to Fig. 19, a photomicrograph (1000x) of unstained live cell preparations of normal diploid fibroblasts **040** exposed to plant TOS **070** (not shown) at approximately two weeks. The experimental procedure for Figs. 18 and 19 comprised applying plant TOS 070 in purified form to cultures of NCl-HI22 non-small cell lung carcinoma **062** or WI38 normal diploid lung fibroblasts **040.** The cells **040** and **062** were incubated in culture medium contain plant TOS **070** for approximately five days. The plant derived TOS **070** induced extensive cell death **060** in the malignant lung cancer cells **062** while displaying no significant cytotoxic effects in the normal lung cells **040** incubated under identical conditions. This observation suggests an important potential clinical therapeutic activity for the plant derived TOS **070.**

The documented tissue transforming properties of TOS have important therapeutic application in the treatment of many other diseases associated with the loss or absence of normal tissue-associated functions, including recessive genetic disorders resulting from single gene mutations such as enzymopathies, relatively common disorders such as cystic fibrosis and muscular dystrophy, and loss of function related to diseases such as diabetes or tissue injury or damage. Treatment of these disorders by exposing the diseased tissues to TOS derived from normal cells of the same tissue type might be expected to induce the morphological and functional reversion of these tissues to a normal tissue type.

### TOS and Clinical Application for Tissue Engineering

Observation indicated that these unique cell-associated structures are highly pleomorphic with respect to their structural parameters and have extraordinarily diverse functionally with respect to their ability to interact with each other and with tumor tissue. Fig. 4 above demonstrates that cell-to-cell attachments are mediated by TOS interactions. High levels of TOS activity and concentration were associated with points of cellular interaction that ultimately became intercellular connections or junctions. The association of TOS with extra-cellular matrix (ECM) fiber structures appeared to tether the TOS to their contact cells to direct their movements in organizing the cell-to-cell associations characteristic of this primitive and fundamental component of solid tumor interactions. Direct entry of the TOS into cells, by what appeared to be membrane fusion followed by re-emergence, was observed to be associated with the activation of the membrane activity and motility of these cells, thus propelling them to interact with neighboring cells to form distinct cell-to-cell contacts.

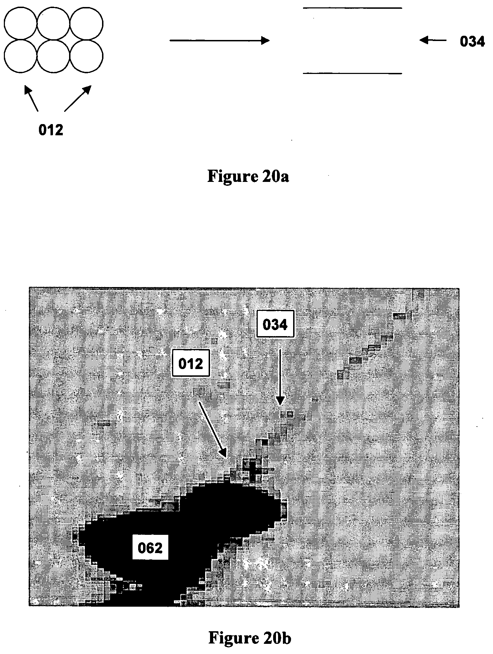

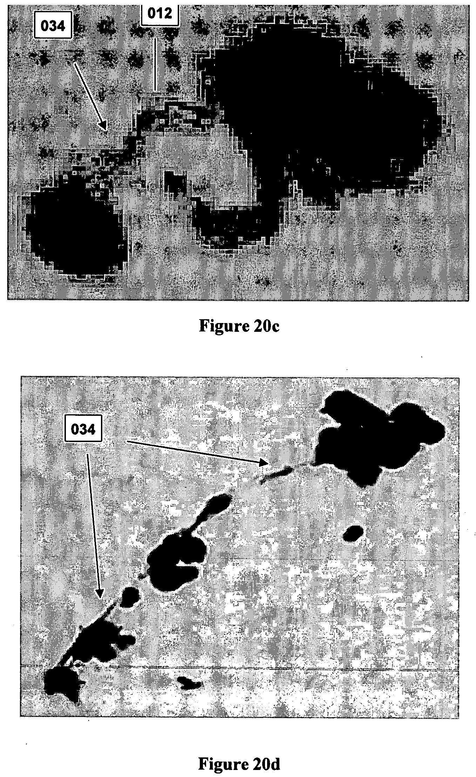

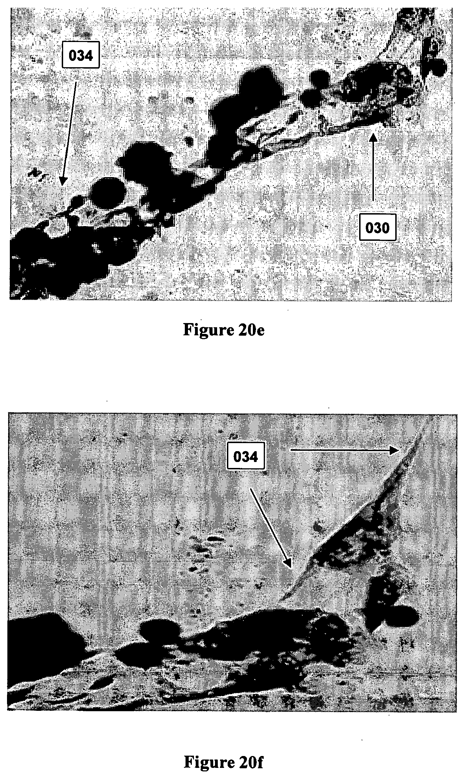

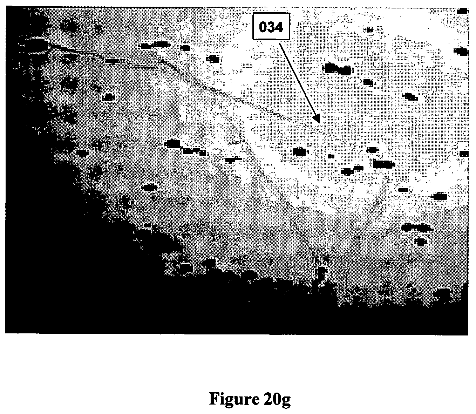

As shown in Figs. 20a - g, the motility of these cells containing TOS **012** propelled them to interact with neighboring cells by utilizing TOS tubules **034** to form distinct cell-to-cell contacts **030.** Fig. 20a is a diagram of an embodiment illustrating formation of TOS tubules **034** from fusion of individual TOS **012.** In Figs. 20b - 20g, non-small cell carcinoma NCl-H1299 **062** was stained with periodic acid/Schiff reagent and photomicrographed (1000x) at day 1 of solid tumor formation. Fig. 20b shows TOS tubule **034** formation by TOS **012** fusion from a small tumor cell cluster **064.** Note the TOS **012** emerging from the cell **062.** Fig. 20c shows early stage tubule **034** formation and TOS bodies **012** clearly evident. Figs. 20d - 20f show TOS tubules **034** joining small clusters of tumor cells **064.** Fig. 20d shows tubules **034** formed from fusing TOS **012** to facilitate interactions between diverse clusters of cells in culture. Fig. 20e shows TOS tubule **034** interface between cell and TOS tubule **034** boundary, and TOS mediated cell connections **030.** Fig. 20f shows TOS tubule **034** formation. Fig. 20g shows an overview of tubules **034** joining distant regions of tumor **064** at early stage formation **032.**

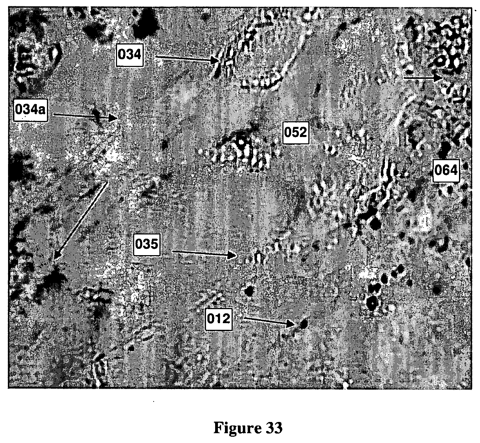

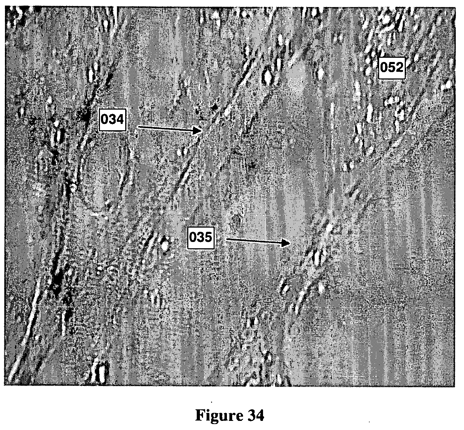

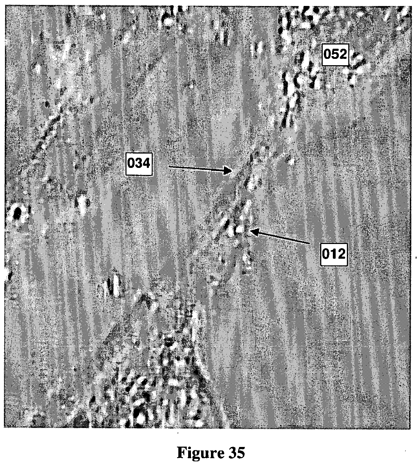

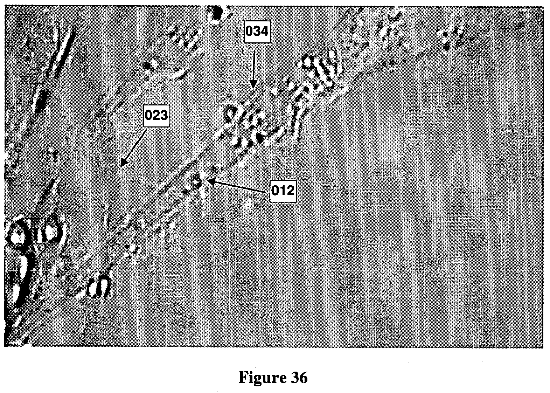

In Fig. 33, a frame from unstained live cell videography, tubules **034** and extensions **035** associated with TOS **012** activity in GBM **052** solid tumor are shown. TOS tubules **034** appear to mediate their formation by fusion at the outer edges of developing tubule **034a.** TOS **012** can also migrate through tubules **034** from one aggregate of cells **064** to another (GBM tumor). In Fig. 34, another a frame from unstained live cell videography, TOS tubules **034** are shown emerging from cell body **052** as major extensions **035** serving as conduits for TOS migration. Fig. 35, another frame from unstained live cell videography, shows cell-to-cell associations mediated by TOS tubule interactions. Note an individual TOS **012** traveling through tubule **034** in GBM **052** tumor. In Fig. 36, another frame from unstained live cell videography, a TOS tubule **034** in association with the cell membrane **023** is shown in GBM **052** tumor cell.

In Fig. 21, TOS movements along ECM fibers produced by the tumor cells appeared to facilitate communication between different clusters of tumor cells associated with their organization to form larger masses via effects on cell mobility and migration. When TOS were observed to interact with patches of tumor cells already linked by cell-to-cell associations, the TOS association promoted the migration of these multi-cellular clusters of tumor cells to merge, thereby generating larger masses of tumor cell aggregates. The association of TOS with tumor cell aggregates appeared to continue until the tumor cells were fully surrounded and in contact with neighboring cells, at which time the TOS frequently were observed to re-enter cells and become quiescent.

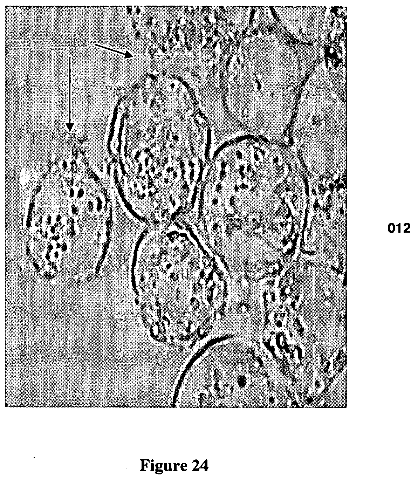

Figs. 21a - i illustrate TOS bodies in solid tumor architecture. Solid tumors of Glioblastoma multiforme DBTRG.05MG were stained with periodic acid/Schiff reagent and photomicrographed (1000x) at 48 days in culture. Fig. 21a shows a GBM cell **062** in association with a peripheral structure formed by TOS **012.** Fig. 21b is an example of highly organized tubule structure **034** formed by TOS **012** embedded in solid tumor. Fig. 21c shows TOS forming **018** in cell membrane of GBM solid tumor spheroid **038.** Fig. 21d displays elements of solid tumor architecture showing highly tubular structures **034** formed by TOS in solid tumor GBM spheroids **038.** Figs. 21e - i illustrate TOS tubules structural details in association with TOS bodies at points of origin and internal structure. Fig. 21e shows solid tumor GBM edge **038** displaying unique morphology correlated with sites of TOS **018** formation. Fig. 21f is another view showing TOS structure **034** formation with solid tumor GBM **038.** Fig. 21g is another view showing highly organized TOS geometric structural **034** formation within solid tumor GBM **038.** Fig. 21h is another view showing TOS structure **034** formation with solid tumor GBM **038.** Fig. 21i is a 4X low magnification of GBM solid tumor **038** in culture used as source of photomicroscopy images shown above at higher magnification. In addition, Fig. 24, a frame from live cell videography, shows TOS - mediated cell movement in NCl-H1299 lung carcinoma cells as indicated by the arrows.

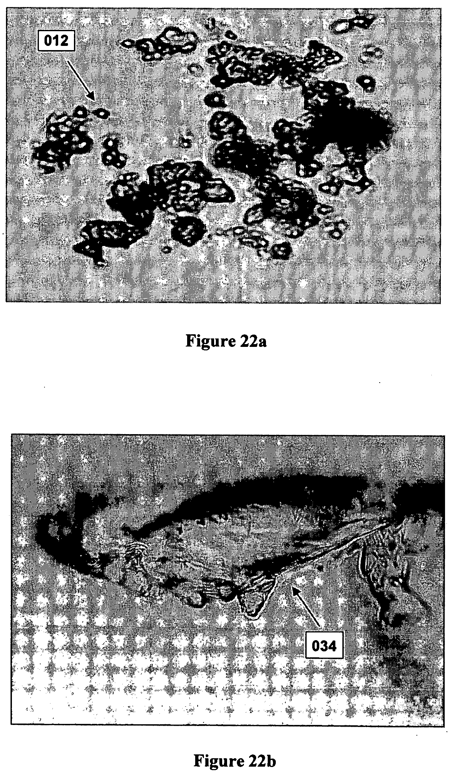

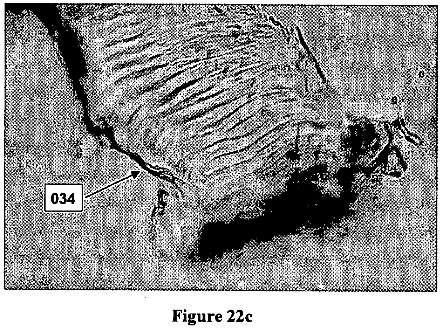

Studies of cell-free isolated TOS showed that these structures have the capability to reproduce and maintain long-term viability in culture as shown in Fig. 22. TOS were observed to generate complex tissue-like structures in vitro under these conditions. TOS structures were also observed to be produced in normal lung cell cultures (WI38) in vitro. Figs. 22a - c are photomicrographs (1000x) showing TOS **012** isolated in culture. Fig. 22a is an H & E stained preparation showing cell free TOS **012** with arrow indicating single particle. Fig. 22b is an unstained photomicrograph shows structures **034** formed by TOS in cell free culture. Fig. 22c shows a scroll-like geometric structure **034** formed by TOS in cell free culture.

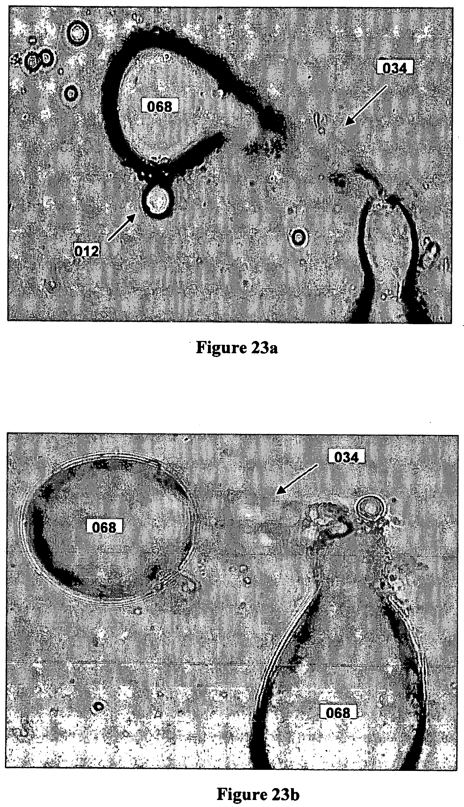

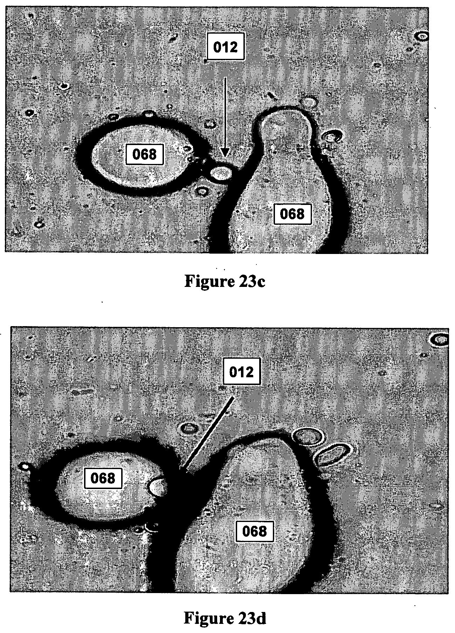

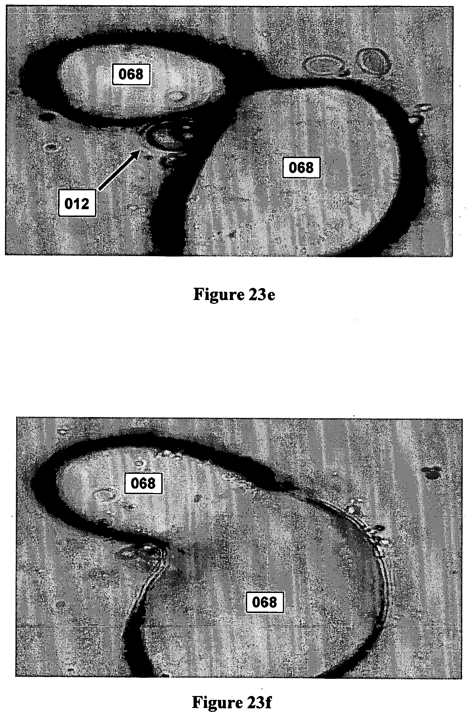

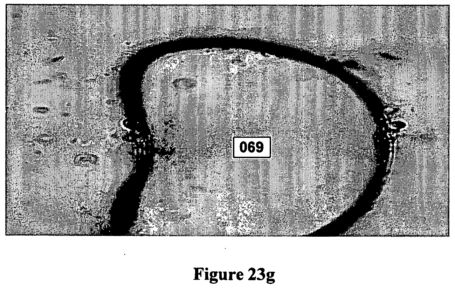

Figure 23 is a series of live imaging photomicrographs (1000x) documenting the activities of cell-free TOS preparation in culture, merging and interacting to form larger structures. In Fig. 23a and 23b, contact between large vesicles **068** formed by prior TOS particle fusion is established by tubular **034** projections. The sequence in Figs. 23c - 23g documents migration of TOS vesicles **068** which facilitates contact, then fusion of vesicles to form one large unit **069.**

By utilizing TOS, an embodiment of a method of tissue engineering or tissue repair may comprise the steps of providing a source of tissue genetic material, culturing the tissue genetic material with an effective tissue organizing structure composition, isolating and purifying the tissue organization structure-tissue genetic material product, culturing the product with tissue target cells, promoting cellular differentiation of said target cells to said tissue specific phenotype activity, the differentiation being effective for said tissue repair.

An additional embodiment for producing cells with a tissue-specific phenotype useful for autogenous tissue repair comprises providing tissue from an intended patient, converting said patient tissue cells into repair/replacement cells by culturing said patient tissue cells with an effective tissue organizing structure composition, isolating and purifying the tissue organization structure-patient tissue product, culturing the product with tissue target cells, promoting cellular differentiation of said target cells to said patient tissue specific phenotype, the converted cells having effective patient tissue-specific phenotype activity.

An additional embodiment fora tissue repair precursor composition comprises a biocompatible matrix, a dense cell mass growing on the matrix and exposed to a culture medium, the dense cell mass being converted tissue-specific phenotype exhibiting cell appropriate morphology, the culture medium including a cellular inducing agent and at least one tissue organizing structure composition that promotes differentiation along the tissue-specific pathway.

### TOS Assessment in Genetic Engineering

TOS mediated genetic exchanges were observed following TOS fusion with tumor cells and were observed to produce rapid and dramatic morphogenetic alterations in tumor cell appearance, including distortions of size, nuclear to cytoplasmic ratio and surface asymmetries that in some cases were associated with the induction of tumor cell fission to generate tumor cells comprised entirely of TOS structures, that we have called 'TOS bags" (see Fig. 3). Tumor cell fission to release large numbers of TOS appeared to be associated with tumor spread as TOS interacted with larger tumor cells, stimulated cell division and frequently were observed to organize to form cylindrical masses of TOS tubules (see Fig. 20).

TOS were observed to exist in one of two forms: either as 'empty" membrane-bound vesicles or as vesicles containing genetic material. The genetic material was observed to be acquired by the empty vesicles following their entry into tumor cells, as this was often followed by their subsequent emergence with newly acquired contents comprised of genetic material.

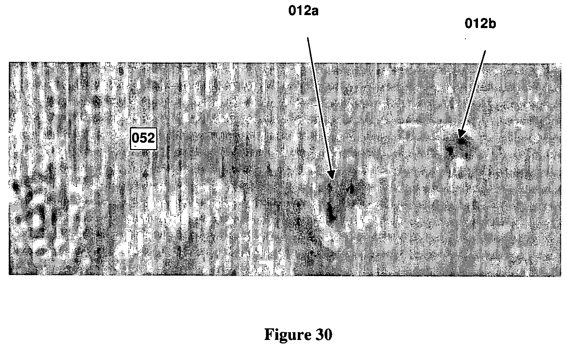

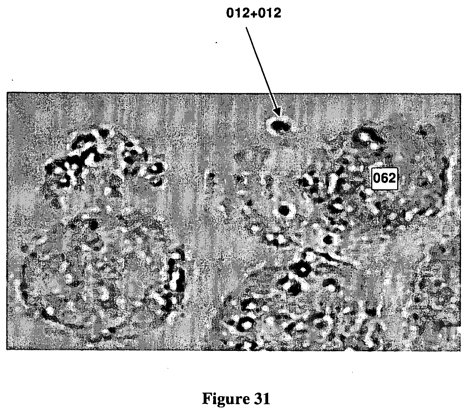

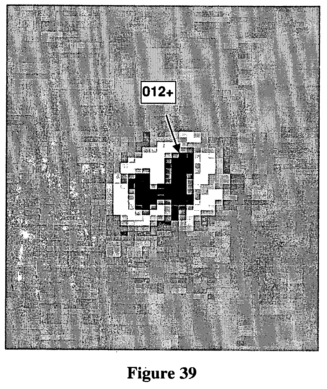

The TOS were also observed to fuse with one another and appeared to exchange genetic material before dissociating and moving apart. Fig. 30, a frame from live cell videography, shows TOS-TOS genetic exchange by fusion of two TOS **012a** and **012b** associated with chromosomal interactions (colon carcinoma cells SW620). In-Fig. 31, a frame from live cell videography, TOS-cell associations with cell migration (NCl-H1299 lung carcinoma) and two TOS **012+012** fused with chromosome interactions are shown. Fig. 39 is a frame from unstained live cell videography showing fused TOS **012+** in genetic exchange. Note densely staining area in center suggesting genetic recombination **056** (MDA_MB_231 breast carcinoma cells).

Overall, TOS were observed to be extraordinarily plastic with respect to their ability to fuse with one another to form doublets or even triplet complexes, and to enter tumor cells. These complexes form transiently and are frequently associated with genetic recombination. This plasticity extends to their genetic contents as well, in that TOS were observed to exchange genetic information with other TOS and also with tumor cells.

The observed activities of TOS demonstrate their potential utility in genetic engineering. TOS have been shown to be capable of inducing stable genetic phenotypic transformation of cultured cells. This capacity could be exploited to introduce therapeutic genes into target recipient cells. Moreover, the observed capacity of TOS to induce rapid morphogenetic conversion at the cellular level implicates these structures as potentially valuable tools to facilitate tissue transformations for therapeutic purposes

It is understood that the embodiments and descriptions herein are merely instruments of the application of the utility of TOS and those skilled in the art should realize that changes may be made without departure from the essential elements and contributions to the art as taught herein.

## Claims

1. A composition comprising at least one tissue organizing structure (TOS), wherein said at least one TOS is a eukaryotic cell-derived, cell-free membrane-delimited vesicle, has a diameter about 2 microns and an interior comprising DNA and is capable of inducing stable morphogenetic transformation of recipient cells, and a diluent.

2. The composition of claim 1, wherein said TOS is of human or plant origin.

3. The composition of claims 1 or 2, wherein the TOS has an interior comprising double stranded DNA.

4. The composition of claims 1, 2 or 3, wherein said at least one TOS (12) is prepared from an origin tissue by the process of:
a. inducing a cancer cell or genetically altered cell (14), wherein said genetically altered cell is comparable to a hyperplastic cell or emerging carcinoma in situ cell, to abrogate stromal detachment in the origin tissue with trypsinization, wherein said induction causes an initiation of changes in the cell membrane comprising a production of at least one TOS bud (18) in said membrane, wherein said TOS bud (18) is a bud on the surface membrane of said cell (14); and
b. releasing at least one TOS (12) from said at least one bud (18).

5. The composition of claims 1, 2 or 3 comprising a plurality of TOSs, wherein said plurality of TOSs is formed by the process of:
a. initiating fusion of a TOS with a cell, wherein said initiation of fusion of the TOS with the cell causes an initiation of intracellular production of a plurality of TOSs; and
b. releasing said plurality of TOSs from said cell by fission.

6. The composition of claims 1, 2 or 3, wherein said at least one TOS is formed and isolated by the process of:
a. stimulating production of TOS in cells by culturing said cells in a serum-free medium;
b. removing said medium;
c. washing said cells with a phosphate buffered saline solution;
d. facilitating accumulation of TOS in said cells by contacting said cells with trypsin for a predetermined period of time to produce trypsanized cells;
e. stimulating release of TOS by contacting said trypsanized cells with a fetal bovine solution of a predetermined concentration;
f. releasing TOS from said trypsanized cells by incubating said trypsantized cells at a predetermined temperature for a predetermined duration in a container; and
g. removing said cells from said container to yield isolated TOS.

7. The composition of claim 6, wherein said at least one isolated TOS is purified by the additional steps of:
h. mechanically removing said isolated TOS from said container; and
i. resuspending said removed TOS in a diluent suitable to maintain TOS activity.

8. The composition of claim 7, wherein said diluent is a type of culture medium.

9. The composition of claim 6, 7 or 8, wherein said cells are diploid fibroblasts (W138).

10. The composition of claim 1, wherein said at least one TOS is formed from a species of plant by the process of:
a. washing a plant material with alcohol;
b. rinsing said plant material with water to yield cleaned plant material;
c. adding a cell culture media having a predetermined concentration of fetal bovine serum to said cleaned plant material;
d. mixing said cell culture media and said cleaned plant material to yield a homogenate;
e. incubating said homogenate at a predetermined temperature for a predetermined duration in a container;
f. removing the media and plant material from said container to yield at least one generally isolated TOS in said container.

11. The composition of claim 10, wherein the concentration of fetal bovine serum in said cell culture media is 10%.

12. The composition of claim 10, wherein said plant material is a species of fern.

13. The composition of claim 1 for use in a therapeutically effective dosage to treat cancer cells selected from the group consisting of leukemia cells, non-small cell lung cancer cells, colon cancer cells, central nervous system cancer cells, melanoma cells, ovarian cancer cells, pancreatic cancer cells, renal cancer cells, prostate cancer cells, breast cancer cells and sarcomas in an individual or animal.

14. An ex vivo or in vitro method of inducing death of a malignant cell, comprising:
a. providing a TOS composition derived from normal functioning cells, wherein said TOS is a cell-derived membrane-delimited vesicle, has a diameter about 2 microns and an interior comprising DNA and is capable of inducing stable morphogenetic transformation of recipient cells;
b. contacting said composition with a culture of malignant cells selected from the group consisting of leukemia cells, non-small cell lung cancer cells, colon cancer cells, central nervous system cancer cells, melanoma cells, ovarian cancer cells, pancreatic cancer cells, renal cancer cells, prostate cancer cells, breast cancer cells and sarcomas; and
c. incubating said culture to induce death of at least one of said malignant cells.

15. An ex vivo or in vitro method of transferring genetic material between cells, comprising:
a. selecting a plurality of cells from a patient, the cells having genetic material that expresses specific phenotype characteristics;
b. transferring said genetic material to a plurality of TOSs by incubating a culture having said selected cells and a composition having TOSs to produce TOS-genetic material, wherein said TOS is a cell-derived membrane-delimited vesicle, has a diameter about 2 microns and an interior comprising DNA and is capable of inducing stable morphogenetic transformation of recipient cells;
c. isolating and purifying said TOS-genetic material from said selected cell culture;
d. suspending said TOS-genetic material in a phosphate buffered saline solution;
e. adding said TOS-genetic material suspension to a culture of target cells;
f. incubating said culture of target cells with said TOS-genetic material suspension to produce transformed target cells comprising the phenotype of said source cells.

## Patentansprüche

1. Zusammensetzung umfassend mindestens eine Gewebeorganisationsstruktur (TOS), wobei besagte mindestens eine TOS ein von einer eukaryotischen Zelle abgeleitetes, zellfreies membranbeschränktes Vesikel ist, einen Durchmesser von etwa 2 Mikrometer und einen DNS umfassenden Innenraum aufweist und in der Lage ist bei Empfängerzellen eine stabile morphogenetische Transformation zu induzieren, und ein Verdünnungsmittel.

2. Zusammensetzung nach Anspruch 1, wobei besagtes TOS humanen oder pflanzlichen Ursprungs ist.

3. Zusammensetzung nach Anspruch 1 oder 2, wobei der TOS-Innenraum doppelsträngige DNS umfasst.

4. Zusammensetzung nach den Ansprüchen 1, 2 oder 3, wobei besagte mindestens eine TOS (12) aus einem Ursprungsgewebe durch folgendes Verfahren hergestellt wird:
a. Induktion einer Krebszelle oder einer genetisch veränderten Zelle (14), wobei besagte genetisch veränderte Zelle vergleichbar ist mit einer hyperplastischen Zelle oder einer entstehenden Karzinom-in-situ-Zelle, um eine Stromaloslösung in dem Ursprungsgewebe mit einer Trypsinierung außer Kraft zu setzen, wobei besagte Induktion eine Einleitung von Veränderungen in der Zellmembran verursacht umfassend die Bildung von mindestens einer TOS-Knospe (18) in besagter Membran, wobei besagte TOS-Knospe (18) eine Knospe an der Oberflächenmembrane der besagten Zelle (14) ist; und
b. Freisetzung von mindestens einer TOS (12) von besagter mindestens einer Knospe (18).

5. Zusammensetzung nach den Ansprüchen 1, 2 oder 3 umfassend eine Vielzahl an TOS, wobei besagte Vielzahl an TOS durch folgendes Verfahren gebildet werden:
a. Induktion einer Fusion von einer TOS mit einer Zelle, wobei besagte Induktion der Fusion der TOS mit der Zelle eine Einleitung einer intrazellulären Bildung einer Vielzahl an TOS verursacht; und
b. Freisetzung von besagter Vielzahl an TOS von besagter Zelle durch Fission.

6. Zusammensetzung nach den Ansprüchen 1, 2 oder 3, wobei besagte mindestens eine TOS durch folgendes Verfahren gebildet und isoliert wird:
a. Stimulation der Bildung von TOS in Zellen durch die Kultivierung besagter Zellen in einem Serum-freien Medium;
b. Entfernung des besagten Mediums;
c. Waschung besagter Zellen mit einer Phosphat-gepufferten Salzlösung;
d. Unterstützung der Akkumulation von TOS in besagten Zellen durch kontaktieren besagter Zellen mit Trypsin über einen vorbestimmten Zeitraum um trypsinierte Zellen zu erhalten;
e. Stimulation der Freisetzung von TOS durch Kontaktieren besagter trypsinierter Zellen mit fötaler Rinderlösung einer vorbestimmten Konzentration;
f. Freisetzung von TOS von besagten trypsinierten Zellen durch Inkubation besagter trypsinierter Zellen bei einer vorbestimmten Temperatur für eine vorbestimmte Zeitdauer in einem Behältnis; und
g. Entfernung besagter Zellen aus besagtem Behältnis um isolierte TOS zu ernten.

7. Zusammensetzung nach Anspruch 6, wobei besagte mindestens eine isolierte TOS durch die folgenden zusätzlichen Schritte gereinigt wird:
h. mechanische Entfernung besagter isolierter TOS aus besagtem Behältnis; und
i. Resuspendierung besagter entfernter TOS in einem Verdünnungsmittel, das in der Lage ist die TOS-Aktivität aufrechtzuerhalten.

8. Zusammensetzung nach Anspruch 7, wobei besagtes Verdünnungsmittel eine Form eines Kulturmediums ist.

9. Zusammensetzung nach Anspruch 6, 7 oder 8, wobei besagte Zellen diploide Fibroblasten sind (W138).

10. Zusammensetzung nach Anspruch 1, wobei besagte mindestens eine TOS von einer Pflanzensorte durch folgendes Verfahren gebildet wird:
a. Waschen eines Pflanzenmaterials mit Alkohol;
b. Abspülen des besagten Pflanzenmaterials mit Wasser um gereinigtes Pflanzenmaterial zu erhalten;
c. Hinzufügung eines Zellkulturmediums mit einer vorbestimmten Konzentration an fötalem Rinderserum zu besagtem gereinigten Pflanzenmaterial;
d. Mischung des besagten Zellkulturmediums und besagten gereinigten Pflanzenmaterials um ein Homogenat zu erhalten;
e. Inkubation des besagten Homogenats bei einer vorbestimmten Temperatur für eine vorbestimmte Zeitdauer in einem Behältnis;
f. Entfernung des Mediums und des Pflanzenmaterials aus besagtem Behältnis um mindestens eine generell isolierte TOS in besagtem Behältnis zu ernten.

11. Zusammensetzung nach Anspruch 10, wobei die Konzentration an fötalem Rinderserum in besagtem Kulturmedium 10% ist.

12. Zusammensetzung nach Anspruch 10, wobei besagtes Pflanzenmaterial eine Farnsorte ist.

13. Zusammensetzung nach Anspruch 1 zur Verwendung in einer pharmazeutisch aktiven Dosis zur Behandlung von Krebszellen ausgewählt aus der Gruppe bestehend aus Leukämiezellen, nicht-kleinzellige Lungenkrebszellen, Darmkrebszellen, Krebszellen des zentralen Nervensystems, Melanomzellen, Eierstockkrebszellen, Bauchspeicheldrüsenkrebszellen, Nierenkrebszellen, Prostatakrebszellen, Brustkrebszellen und Sarkomas bei einem Individuum oder Tier.

14. Ex vivo oder in vitro Verfahren zur Induktion des Todes einer bösartigen Zelle, umfassend:
a. Bereitstellung einer von normal funktionierenden Zellen abgeleiteten TOS-Zusammensetzung, wobei besagte TOS ein zellabgeleitetes membranbeschränktes Vesikel ist, einen Durchmesser von etwa 2 Mikrometer und einen DNS umfassenden Innenraum aufweist und in der Lage ist bei Empfängerzellen eine stabile morphogenetische Transformation zu induzieren;
b. Kontaktieren besagter Zusammensetzung mit einer Kultur von bösartigen Zellen ausgewählt aus der Gruppe bestehend aus Leukämiezellen, nicht-kleinzellige Lungenkrebszellen, Darmkrebszellen, Krebszellen des zentralen Nervensystems, Melanomzellen, Eierstockkrebszellen, Bauchspeicheldrüsenkrebszellen, Nierenkrebszellen, Prostatakrebszellen, Brustkrebszellen und Sarkomas; und
c. Inkubation besagter Kultur um den Tod von mindestens einer der besagten bösartigen Zellen zu induzieren.

15. Ex vivo oder in vitro Verfahren zur Übertragung von genetischen Material zwischen Zellen, umfassend:
a. Selektion einer Vielzahl an Zellen von einem Patienten, wobei die Zellen genetisches Material haben, das spezifische phänotypische Eigenschaften exprimiert;
b. Übertragung des besagten genetischen Materials auf eine Vielzahl an TOS durch Inkubation einer Kultur enthaltend selektierte Zellen mit einer TOS enthaltenden Zusammensetzung um TOS-genetisches Material herzustellen, wobei besagte TOS ein zellabgeleitetes membranbeschränktes Vesikel ist, einen Durchmesser von etwa 2 Mikrometer und einen DNS umfassenden Innenraum aufweist und in der Lage ist bei Empfängerzellen eine stabile morphogenetische Transformation zu induzieren;
c. Isolierung und Reinigung des besagten TOS-genetischen Materials von besagter selektierter Zellkultur;
d. Suspendierung des besagten TOS-genetischen Materials in einer Phosphat-gepufferten Salzlösung;
e. Hinzufügung der besagten TOS-genetischen Materialsuspension zu einer Kultur von Zielzellen;
f. Inkubation besagter Kultur von Zielzellen mit besagter TOS-genetischen Materialsuspension um transformierte Zielzellen zu erhalten, die den Phänotyp der besagten Ursprungszellen umfassen.

## Revendications

1. Composition comprenant au moins une structure d'organisation d'un tissu (TOS), où ladite au moins une TOS est une vésicule délimitée par une membrane acellulaire, dérivée d'une cellule eucaryote, possède un diamètre d'environ 2 microns et un espace intérieur comprenant de l'ADN, et est capable d'induire une transformation morphogénétique stable de cellules receveuses, et un diluant.

2. Composition selon la revendication 1, dans laquelle ladite TOS est d'origine humaine ou végétale.

3. Composition selon les revendications 1 ou 2, dans laquelle la TOS possède un espace intérieur comprenant de l'ADN double brin.

4. Composition selon les revendications 1, 2 ou 3, dans laquelle ladite au moins une TOS (12) est préparée à partir d'un tissu d'origine par le procédé consistant à :
a. induire une cellule cancéreuse ou une cellule génétiquement altérée (14), où ladite cellule génétiquement altérée est comparable à une cellule hyperplasique ou à une cellule de carcinome *in situ* émergente, pour abroger le détachement stromal dans le tissu d'origine par une trypsinisation, où ladite induction provoque une initiation de modifications dans la membrane cellulaire comprenant une production d'au moins un bourgeon de TOS (18) dans ladite membrane, où ledit bourgeon de TOS (18) est un bourgeon sur la membrane de surface de ladite cellule (14) ; et
b. libérer au moins une TOS (12) à partir dudit au moins un bourgeon (18).

5. Composition selon les revendications 1, 2 ou 3 comprenant une pluralité de TOS, où ladite pluralité de TOS est formée par le procédé consistant à :
a. initier la fusion d'une TOS avec une cellule, où ladite initiation de la fusion de la TOS avec la cellule provoque une initiation de la production intracellulaire d'une pluralité de TOS ; et
b. libérer ladite pluralité de TOS à partir de ladite cellule par fission.

6. Composition selon les revendications 1, 2 ou 3, dans laquelle au moins une TOS est formée et isolée par le procédé consistant à :
a. stimuler la production d'une TOS dans des cellules par la mise en culture desdites cellules dans un milieu exempt de sérum ;
b. éliminer ledit milieu ;
c. laver lesdites cellules avec une solution saline tamponnée au phosphate ;
d. faciliter l'accumulation d'une TOS dans lesdites cellules par la mise en contact desdites cellules avec de la trypsine pendant une période prédéterminée afin de produire des cellules trypsinisées ;
e. stimuler la libération de TOS par la mise en contact desdites cellules trypsinisées avec une solution de sérum bovin foetal ayant une concentration prédéterminée ;
f. libérer une TOS à partir desdites cellules trypsinisées par l'incubation desdites cellules trypsinisées à une température prédéterminée pendant une durée prédéterminée dans un récipient ; et
g. retirer lesdites cellules dudit récipient pour générer une TOS isolée.

7. Composition selon la revendication 6, dans laquelle ladite au moins une TOS isolée est purifiée par les étapes supplémentaires consistant à :
h. mécaniquement retirer ladite TOS isolée dudit récipient ; et
i. remettre en suspension ladite TOS retirée dans un diluant approprié pour maintenir l'activité de la TOS.

8. Composition selon la revendication 7, où ledit diluant est un type de milieu de culture.

9. Composition selon la revendication 6, 7 ou 8, où lesdites cellules sont des fibroblastes diploïdes (W138).

10. Composition selon la revendication 1, dans laquelle ladite au moins une TOS est formée à partir d'une espèce de plante par le procédé consistant à :
a. laver un matériel végétal avec de l'alcool ;
b. rincer ledit matériel végétal avec de l'eau pour générer un matériel végétal nettoyé ;
c. ajouter un milieu de culture cellulaire ayant une concentration prédéterminée de sérum bovin foetal audit matériel végétal nettoyé ;
d. mélanger ledit milieu de culture cellulaire et ledit matériel végétal nettoyé pour générer un homogénat ;
e. incuber ledit homogénat à une température prédéterminée pendant une durée prédéterminée dans un récipient ;
f. retirer le milieu et le matériel végétal dudit récipient pour générer au moins une TOS généralement isolée dans ledit récipient.

11. Composition selon la revendication 10, où la concentration du sérum bovin foetal dans ledit milieu de culture cellulaire est de 10 %.

12. Composition selon la revendication 10, où ledit matériel végétal est une espèce de fougère.

13. Composition selon la revendication 1 destinée à être utilisée en une dose thérapeutiquement efficace pour traiter des cellules cancéreuses sélectionnées dans le groupe consistant en des cellules de la leucémie, des cellules du cancer du poumon non à petite cellules, des cellules du cancer du côlon, des cellules du cancer du système nerveux central, des cellules de mélanome, des cellules du cancer de l'ovaire, des cellules du cancer du pancréas, des cellules du cancer du rein, des cellules du cancer de la prostate, des cellules du cancer du sein et des sarcomes chez un individu ou un animal.

14. Procédé *ex vivo* ou *in vitro* d'induction de la mort d'une cellule maligne, comprenant :
a. la mise à disposition d'une composition de TOS dérivée de cellules fonctionnant normalement, où ladite TOS est une vésicule délimitée par une membrane dérivée d'une cellule, possède un diamètre d'environ 2 microns et un espace intérieur comprenant de l'ADN, et est capable d'induire une transformation morphogénétique stable de cellules receveuses ;
b. la mise en contact de ladite composition avec une culture de cellules malignes sélectionnées dans le groupe consistant en des cellules de la leucémie, des cellules du cancer du poumon non à petite cellules, des cellules du cancer du côlon, des cellules du cancer du système nerveux central, des cellules de mélanome, des cellules du cancer de l'ovaire, des cellules du cancer du pancréas, des cellules du cancer du rein, des cellules du cancer de la prostate, des cellules du cancer du sein et des sarcomes ; et
c. l'incubation de ladite culture pour induire la mort d'au moins une desdites cellules malignes.

15. Procédé *ex vivo* ou *in vitro* de transfert de matériel génétique entre des cellules, comprenant :
a. la sélection d'une pluralité de cellules à partir d'un patient, les cellules possédant du matériel génétique qui exprime des caractéristiques d'un phénotype spécifique ;
b. le transfert dudit matériel génétique dans une pluralité de TOS par l'incubation d'une culture comportant lesdites cellules sélectionnées et d'une composition comportant des TOS afin de produire un matériel génétique de TOS, où ladite TOS est une vésicule délimitée par une membrane dérivée d'une cellule, possède un diamètre d'environ 2 microns et un espace intérieur comprenant de l'ADN, et est capable d'induire une transformation morphogénétique stable de cellules receveuses ;
c. l'isolement et la purification dudit matériel génétique de TOS à partir de ladite culture cellulaire sélectionnée ;
d. la mise en suspension dudit matériel génétique de TOS dans une solution saline tamponnée au phosphate ;
e. l'ajout de ladite suspension de matériel génétique de TOS à une culture de cellules cibles ;
f. l'incubation de ladite culture de cellules cibles avec ladite suspension de matériel génétique de TOS afin de produire des cellules cibles transformées comprenant le phénotype desdites cellules sources.
